# FASCICULE DE BREVET EUROPEEN

(11) **EP 0 720 974 B1**
(45) Date de publication et mention de la délivrance du brevet: **27.11.2002**
(21) Numéro de dépôt: 96400037.6
(22) Date de dépôt: 08.01.1996
(51) Int. Cl.: C05F 11/08, A01N 63/00

(54) **Complexes bactériens et leurs applications au traitement des résidus d'origine biologique**
Bakterielle Mittel und seine Verwendung zur Behandlung von biologischen Abfällen
Bacterial preparation and its use for treating wastes of biological origin

(30) Priorité: 09.01.1995 FR 9500162
(43) Date de publication de la demande: 10.07.1996
(73) Titulaire: COBIOTEX, 35135 Rennes-Chantepie (FR)
(72) Inventeur: Penaud, Jean, F-37000 Tours (FR)
(74) Mandataire: Orès, Bernard

(56) Documents cités:
- DATABASE WPI Section Ch, Week 8416 Derwent Publications Ltd., London, GB; Class C03, AN 84-098739 & JP-B-59 013 175 ( FUKUMOTO T) , 28 Mars 1984
- DATABASE WPI Section Ch, Week 9317 Derwent Publications Ltd., London, GB; Class C04, AN 93-140524 & JP-A-05 078 663 ( KATAYAMA A) , 30 Mars 1993
- DATABASE WPI Section Ch, Week 8509 Derwent Publications Ltd., London, GB; Class D15, AN 85-053996 & JP-A-60 012 198 ( UCHIMIZU M) , 22 Janvier 1985
- DATABASE WPI Section Ch, Week 8940 Derwent Publications Ltd., London, GB; Class D13, AN 89-243082 & JP-A-01 211 487 ( CALPIS FOOD IND KK) , 24 Août 1989
- DATABASE WPI Section Ch, Week 9336, Derwent Publications Ltd., London, GB; Class D16, AN 1993-282810, XPDW: AN 93- & HU 63 189 A (CSORNAI A) 28 Juillet 1993
- DATABASE WPI Section Ch, Week 9331 Derwent Publications Ltd., London, GB; Class C03, AN 93-247555 & JP-A-05 168 455 ( HIGA T) , 2 Juillet 1993
- DATABASE WPI Section Ch, Week 8429 Derwent Publications Ltd., London, GB; Class D15, AN 84-179097 & JP-A-59 098 799 ( TAISO KK) , 7 Juin 1984

## Description

La présente invention est relative à des complexes bactériens, aptes à être utilisés dans la digestion, la décomposition et la transformation des résidus d'origine biologique sous forme de biomasse et de composés organiques stables et non polluants et à leurs applications au traitement des déchets d'origine biologique, tels que déjections (litières de porcs, de ruminants, d'équidés ou de volailles ou lisiers, déjections humaines) ou purins, cadavres, eaux stagnantes et leur transformation en compost ou autres composés azotés stables, biodégradables et non polluants.

Les organismes hétérotrophes utilisent les composés azotés, dont les protéines végétales ou animales, comme sources nutritives et restituent l'azote à la terre par leurs excréta ou par les produits de décomposition post-mortem, principalement sous forme d'ammoniaque ou d'urée qui sont transformés en nitrites et en nitrates par des bactéries nitrifiantes présentes dans le sol, telles que *Nitrosomonas* ou *Nitrobacter.*

Or, une production importante de composés azotés sous forme gazeuse ou liquido-solide, tels que ammoniac, ammoniaque, nitrites et nitrates, constitue une cause importante de pollution atmosphérique, des sols, des cours d'eau et des nappes phréatiques. Tous ces processus de destruction entraînent la formation de composés d'odeurs particulièrement nauséabondes (NH₃, H₂S notamment...)

On connaît le Brevet JP-B-59 013175 qui vise la fermentation anaérobie d'un fourrage et décrit la préparation de fourrage par fermentation de paille sèche avec un *Baclllus (Baclllus subtilis)* et des *Lactobacillus (Lactobacillus plantarum* et *Lactobacillus brevis).*

On connaît également la Demande de Brevet JP-A-05078663 qui cherche à obtenir une composition bonifiant les sols et répulsive vis-à-vis des taupes, et décrit l'obtention d'une composition bonifiant les sols par inoculation d'une préparation à base de soja à l'aide, d'une part, d'une culture de *Clostridium butyricum, de Bacillus cereus, de Lactobacillus casei subsp. casei et de Lactobacillus fermentum,* et d'autre part, d'une culture de *Bacillus cereus,* de *Lactobacillus casei subsp. phamnosus, de Lactobacillus acidophiles et d'Acetobacter aceti.*

On connaît, en outre, des procédés de traitement des lisiers, des litières ou d'eaux résiduaires, qui font intervenir une culture bactérienne et des enzymes, ou une culture bactérienne, des enzymes et des levures, notamment pour diminuer les dégagements d'ammoniac et les odeurs, particulièrement au moment des manipulations et de l'épandage (lisier). De telles compositions contiennent en particulier comme bactérie : *Bacillus subtilis,* comme enzymes : batinase et amylase et comme levures : *Saccharomyces cerevisiae* (Demande de Brevet français 2 658 077).

Toutefois, les compositions connues pour traiter les déjections ne permettent pas la transformation de l'azote minéral (NH₄, NO₂ et NO₃) et des urates, en aminoacides et protéines (azote organique), mais ne font que diminuer les dégagements d'ammoniac, par adsorption ou solubilisation. De telles transformations n'entraînent pas une réorganisation de l'azote, par son utilisation au niveau du métabolisme azoté des micro-organismes.

En conséquence, un traitement efficace des produits d'origine biologique est nécessaire et crucial pour permettre la transformation de l'azote minéral (NH₄, NO₂ et NO₃) et des urates en azote organique (aminoacides et protéines), pour éviter la pollution et remettre les déchets dans le circuit de l'anabolisme.

Pour résoudre ce problème, la Demanderesse a sélectionné des complexes bactériens, qui permettent essentiellement la transformation de l'azote minéral en azote organique, sous la forme de protéines bactériennes (stabilisation de l'azote et augmentation de la biomasse), c'est-à-dire qui permettent la transformation des déjections, en composés azotés (compost et/ou composés azotés stables) et, particulièrement pour les déchets ayant un rapport C/N suffisant (en relation avec le taux de matière sèche), en composés riches en acides fulviques et humiques, et non polluants, par digestion et transformation des déjections, tout en éliminant les germes pathogènes associés, notamment *Clostridium*, *Bacteroides,* colibacilles, *Listeria,* salmonelles et staphylocoques.

La présente invention a pour objet un complexe bactérien, caractérisé en ce qu'il comprend au moins un *Bacillus* non pathogène sélectionné dans le groupe constitué par *B. subtilis, B. amyloliquefaciens, B. megaterium, B. licheniformis et B. circulans* et au moins un *Lactobacillus* non pathogène sélectionné dans le groupe constitué par *L. rhamosus, L. paracasei, L. fermentum* et *L. acidophilus,* en ce qu'il utilise en tant que source d'azote, essentiellement de l'azote minéral, notamment l'ammoniaque, les nitrates, les nitrites et des molécules d'azote organique telles que l'urée, les urates, les aminoacides, les bases azotées ou tout autre composé azoté de faible poids moléculaire et en ce qu'il est apte à transformer ledit azote en azote organique, sous la forme de protéines bactériennes.

Conformément à l'invention, ledit complexe bactérien présente au moins les activités enzymatiques suivantes : activité cellulolytique, activité protéolytique, activité amylolytique, activité lipolytique, activité pectinolytique.

De manière inattendue, la combinaison *Bacillus*/*Lactobacillus,* présente dans le complexe bactérien selon l'invention agit en synergie pour :
. utiliser à la fois de l'azote minéral et de l'azote organique,
. resynthétiser des protéines bactériennes et
. éliminer les micro-organismes pathogènes associés aux produits à traiter, tels que *Clostridium, Bacteroides,* colibacilles, *Listeria,* salmonelles et staphylocoques.

Un tel complexe bactérien permet donc effectivement de remettre les déchets dans le circuit de l'anabolisme (organisation en chaîne trophique).

Conformément à l'invention, les proportions *Lactobacillus:Bacillus,* dans ledit complexe, sont comprises, selon les cas, soit entre 100:1 et 1:100, de préférence entre 10:1 et 1:10, soit de 1:1.

Conformément à l'invention, les concentrations en bactéries sont comprises entre 10² et 10⁸ ufc/g.

Ledit complexe bactérien comprend avantageusement au moins un *Bacillus,* à une concentration comprise entre 10² et 10⁷ ufc/g et au moins un *Lactobacillus,* à une concentration comprise entre 10³ et 10⁸ ufc/g. Un tel complexe bactérien trouve, de préférence application au traitement des litières de ruminants, d'équidés ou de porcs.

Selon une disposition avantageuse dudit mode de réalisation, ledit complexe bactérien comprend au moins *B. subtilis* à une concentration comprise entre 10² et 10⁷ ufc/g et un *Lactobacillus* sélectionné dans le groupe constitué par *L. rhamnosus, L. paracasei, L. fermentum*, et *L. acidophilus,* à une concentration comprise entre 10³ et 10⁸ ufc/g.

Selon une autre disposition avantageuse dudit complexe, il comprend les 5 *Bacillus* suivants : *B. subtilis, B. amyloliquefaciens, B. megaterium, B. licheniformis* et *B. circulans,* chacun à une concentration comprise entre 10² et 10⁷ ufc/g et les 4 *Lactobacillus* suivants : *L. rhamnosus, L. paracasei, L. fermentum, L. acidophilus,* chacun à une concentration comprise entre 10³ et 10⁸ ufc/g.

De manière avantageuse, lorsque ledit complexe bactérien comprend plusieurs *Bacillus* et/ou plusieurs *Lactobacillus,* les différentes souches d'un même genre (*Bacillus* ou *Lactobacillus*) sont dans un rapport compris entre 1:1 et 1:100.

Dans un autre mode de réalisation dudit complexe bactérien, il comprend, de préférence, au moins un *Bacillus* utilisant comme source d'azote, les urates, notamment *B. subtilis* à une concentration minimale de 10³ ufc/g, éventuellement un autre *Bacillus,* sélectionné dans le groupe constitué par *B. amyloliquefaciens, B. megaterium, B. licheniformis* et *B. circulans* et au moins un *Lactobacillus* sélectionné dans le groupe constitué par *L. rhamnosus, L. paracasei, L. fermentum, L. acidophilus, à* une concentration comprise entre 10² et 10⁸ ufc/g. Un tel complexe bactérien est particulièrement bien adapté au traitement des litières de volailles ou d'autres monogastriques (à l'exception du porc).

Dans un autre mode de réalisation dudit complexe bactérien, il comprend, de préférence, au moins un *Lactobacillus* sélectionné dans le groupe constitué par *L. rhamnosus*, *L. paracasei, L. fermentum, L. acidophilus* et au moins un *Bacillus* sélectionné dans le groupe constitué par *B. subtilis, B. amyloliquefaciens, B. megaterium, B. licheniformis* et *B. circulans,* le rapport *Lactobacillus:Bacillus* étant compris entre 1:1 et 1:10. Un tel complexe bactérien est particulièrement bien adapté au traitement des lisiers, des lagunes ou des fosses septiques.

Dans un autre mode de réalisation dudit complexe bactérien, il comprend, de préférence, les 2 *Bacillus* suivants : *B. subtilis* et *B. megaterium,* chacun à une concentration comprise entre 10² et 10⁷ ufc/g et au moins un *Lactobacillus* sélectionné dans le groupe constitué par *L. rhamnosus, L. paracasei, L. fermentum, L. acidophilus,* à une concentration comprise entre 10³ et 10⁸ ufc/g. Un tel complexe bactérien est notamment particulièrement bien adapté au traitement des cadavres d'animaux.

De manière surprenante, l'association d'au moins un *Bacillus* et d'au moins un *Lactobacillus,* tels que définis ci-dessus, permet effectivement d'obtenir un complexe bactérien qui :
- transforme des résidus fécaux, urinaires ou autres déchets d'origine biologique, en protéines bactériennes, par voie de synthèse bactérienne, c'est-à-dire en utilisant l'azote minéral provenant des différents déchets, soit directement, soit par dégradation ;
- désodorise le fumier ;
- peut dénitrifier des milieux divers et/ou de l'eau ;
- accélère de manière significative le compostage, sans destruction de la matière et à basses températures (inférieures à 45°C).

Une telle sélection de souches n'entraîne, en outre, pas de perte d'azote au cours du temps (stabilisation du taux d'azote).

En outre, le complexe bactérien conforme à l'invention est stable au cours du temps.

En variante, ledit complexe bactérien comprend au moins un *Bacillus* non pathogène, au moins un *Lactobacillus* non pathogène et un *Pediococcus* non pathogène.

Lorsqu'un complexe bactérien conforme à l'invention comprend un *Pediococcus,* ce dernier est aux mêmes concentrations que les *Lactobacillus.*

De manière surprenante, un tel complexe bactérien présente, outre les propriétés exposées ci-dessus, une activité inhibitrice vis-à-vis de *Staphylococcus aureus.*

La présente invention a également pour objet un complexe bactérien comprenant des souches de *Bacillus* à Gram +, qui présentent les caractéristiques suivantes :
- en ce qu'elles sont susceptibles d'utiliser comme source d'azote, de l'azote minéral, notamment l'ammoniaque, les nitrates, les nitrites et des molécules d'azote organique telles que l'urée, l'urate, les amino-acides, les bases azotées ou d'autres composés azotés de faible poids moléculaire,
- en ce qu'elles présentent au moins l'une des activités suivantes : activité amylolytique, activité cellulolytique, activité lignocellulolytique, activité pectinolytique, activité lipolytique, activité protéolytique, activité kératolytique, activité de type bactériocine ou bactériocine-like,
- en ce qu'elles présentent au moins les caractéristiques biochimiques suivantes : gélatinase +, catalase +, uréase -, oxydase -, indole -, et
- en ce qu'elles sont de préférence aérobies/anaérobies facultatives.

De telles souches de *Bacillus* sont, de préférence, sélectionnées dans le groupe constitué par *Bacillus subtilis*, *Bacillus amyloliquefaciens, Bacillus megaterium, Bacillus licheniformis, Bacillus circulans.*

Lesdites souches de *Bacillus* ont été déposées auprès de la Collection Nationale de Cultures de Microorganismes (CNCM), en date du 8 juin 1994, pour ce qui concerne *Bacillus subtilis,* sous les numéros I-1433, I-1438 et I-1440, pour ce qui concerne *Bacillus amyloliquefaciens,* sous les numéros I-1434 et I-1435, pour ce qui concerne *Bacillus megaterium,* sous le numéro I-1436, pour ce qui concerne *Bacillus licheniformis* sous le numéro I-1437 et pour ce qui concerne *Bacillus circulans,* sous le numéro I-1439.

La présente invention a également pour objet un complexe bactérien comprenant des souches de *Lactobacillus* à Gram +, qui présentent les caractéristiques suivantes :
- en ce qu'elles sont susceptibles d'utiliser comme source d'azote, de l'azote minéral, notamment l'ammoniaque, les nitrates, les nitrites et des molécules d'azote organique telles que l'urée, l'urate, les amino-acides, les bases azotées ou d'autres composés azotés de faible poids moléculaire,
- en ce qu'elles présentent au moins une activité de type bactériocine ou bactériocine-like,
- en ce qu'elles présentent avantageusement au moins les caractéristiques biochimiques suivantes : catalase -, oxydase - et
- en ce qu'elles sont de préférence aérobies/anaérobies facultatives.

De telles souches de *Lactobacillus* sont, de préférence, sélectionnées dans le groupe constitué par *Lactobacillus rhamnosus, Lactobacillus paracasei, Lactobacillus acidophilus* et *Lactobacillus fermentum.*

Lesdites souches de *Lactobacillus* ont été déposées auprès de la Collection Nationale de Cultures de Micro-organismes (CNCM), en date du 28 juillet 1994, pour ce qui concerne *Lactobacillus rhamnosus* sous les numéros I-1450, I-1452, I-1453, I-1454, I-1455, I-1456, I-1459, pour ce qui concerne *Lactobacillus paracasei,* sous les numéros I-1451, I-1457 et I-1458, pour ce qui concerne *Lactobacillus acidophilus,* sous le numéro I-1460, et pour ce qui concerne *Lactobacillus fermentum,* sous le numéro I-1461.

La présente invention a également pour objet un complexe bactérien comprenant des souches de *Pediococcus* à Gram positif, qui présentent les caractéristiques suivantes :
- en ce qu'elles présentent une activité de type bactériocine ou bactériocine-like au moins vis-à-vis de *Staphylococcus aureus,*
- en ce qu'elles présentent avantageusement au moins les caractéristiques biochimiques suivantes : catalase -, oxydase - et
- en ce qu'elles sont de préférence aérobies/anaérobies facultatives.

On peut notamment citer comme souche de *Pediococcus* répondant à cette définition, la souche *Pediococcus pentosaceus*, déposée auprès de la Collection Nationale de Cultures de Micro-organismes (CNCM), en date du 22 décembre 1995, sous le numéro I-1654.

De manière inattendue, des complexes bactériens comprenant en combinaison au moins un *Bacillus* et au moins un *Lactobacillus* et éventuellement *un Pediococ* *cus*, présentant les caractéristiques spécifiées ci-dessus, agissent en synergie pour rendre les produits traités en leur présence, sous une forme utile et réduisent ainsi les inconvénients, gênes, voire pollution desdits produits, à des niveaux sanitairement acceptables pour les sols, les cultures et l'ensemble de la chaîne alimentaire et participent donc favorablement au traitement des déchets.

De manière avantageuse, le complexe bactérien selon l'invention est obtenu à partir desdites souches, comme suit :
- culture et production de chaque souche séparément,
- obtention de différentes cultures ayant chacune des concentrations en micro-organisme de l'ordre de 10¹⁰-10¹¹ ufc/g,
- lyophilisation de chaque culture,
- dilution, entre 1/100-1/1 000 000, de chacune desdites souches lyophilisées, en présence d'un diluant neutre (végétal ou minéral tel que l'argile, lithothamne-grit de maïs...), et
- mélange des différentes souches ainsi diluées pour obtenir le complexe bactérien recherché.

Il peut, en outre, comprendre des additifs tels qu'un traceur chimique ou microbiologique.

La présente invention a également pour objet une composition de traitement de résidus biologiques (produit dilué prêt-à-l'emploi), caractérisée en ce qu'elle comprend en association un complexe bactérien conforme à l'invention, au moins un diluant neutre et au moins un fixateur de microparticules.

En effet, les complexes bactériens, tels que définis ci-dessus, constituent un concentré bactérien qui peut être, de préférence, fixé sur un support pour être utilisé pour le traitement des fumiers, lisiers, composts, cadavres....

De telles compositions comprennent donc avantageusement un diluant neutre, identique ou différent de celui utilisé pour préparer le complexe bactérien, et un fixateur de microparticules, tel que de la mélasse, qui joue à la fois le rôle d'élément énergétique et apporte un effet de collage des particules, des dérivés de l'amidon ou d'autres sucres (sucres complexes à dégradation lente), une huile ou une graisse d'enrobage.

De manière préférée, ladite composition comprend essentiellement 5-15 % de complexe bactérien, 80-89 % de diluant neutre et 3-5 % de fixateur de microparticules, de préférence 10 % de complexe bactérien, 87 % de diluant neutre et 3 % de fixateur de particules.

Ladite composition de traitement présente les mêmes applications que le complexe bactérien conforme à l'invention, à des concentrations inférieures mais efficaces.

Elle peut notamment être utilisée, par incorporation directe dans des litières, des lisiers ou tout autre produit organique à traiter [fumiers, déchets végétaux, déchets organiques (cadavres et déchets des abattoirs)], dans des proportions de 0,2 à 50 kg par tonne de produit organique.

Conformément à l'invention, les proportions de ladite incorporation varient selon l'application, en particulier :
- en ce qui concerne les litières : un complexe bactérien ou une composition de traitement conforme à l'invention, est incorporé(e) à la litière, à raison de 10 kg/t de paille ; un entretien hebdomadaire est préconisé à raison de 3 kg/t de paille ;
- en ce qui concerne les lisiers : un complexe bactérien ou une composition de traitement conforme à l'invention, est incorporé(e) aux lisiers, à raison de 100 g à 1 kg/t ; un entretien hebdomadaire est préconisé à raison de 100 g à 3 kg/t ;
- en ce qui concerne les fumiers à composter : un complexe bactérien ou une composition de traitement conforme à l'invention, est incorporé(e) aux fumiers à composter, à raison de 1 à 5 kg/t, en 1 fois ;
- en ce qui concerne les cadavres à composter : un complexe bactérien ou une composition de traitement conforme à l'invention, est incorporé(e) aux cadavres à composter, à raison de 1 à 10 kg/t, en 1 fois ;
- en ce qui concerne les lagunes : un complexe bactérien ou une composition de traitement conforme à l'invention, est incorporé(e) aux lagunes, à raison de 500 g/m², 1 fois tous les 3 mois ;
- en ce qui concerne les fosses septiques : un complexe bactérien ou une composition de traitement conforme à l'invention, est incorporé(e) aux fosses septiques, à raison de 100 g à 1 kg/t ; un entretien hebdomadaire est préconisé à raison de 100 g à 3 kg/t
- en ce qui concerne la désinfection des locaux et notamment la désinfection des lieux d'aisance : un complexe bactérien ou une composition de traitement, conforme à l'invention, est appliqué(e) sous la forme d'une couche (biofilm artificiel), sur les parois et les sols desdits locaux, à raison de 10 g/l d'eau ; la solution obtenue est de préférence pulvérisée sur lesdites parois.

La présente invention a également pour objet un procédé de traitement de résidus biologiques, tels que les déjections, le fumier, les cadavres ou autres, caractérisé en ce qu'il comprend la mise en contact d'un complexe bactérien conforme à l'invention ou d'une composition telle que définie ci-dessus, avec un résidu biologique à traiter.

Outre les dispositions qui précèdent, l'invention comprend des exemples de mise en oeuvre du procédé objet de la présente invention, donnés uniquement à titre d'illustration de l'objet de l'invention.

**EXEMPLE 1 : Caractéristiques des *Bacillus* conformes à l'invention :** lesdites souches présentent les caractéristiques morphologiques et biochimiques illustrées aux Tableaux suivants :

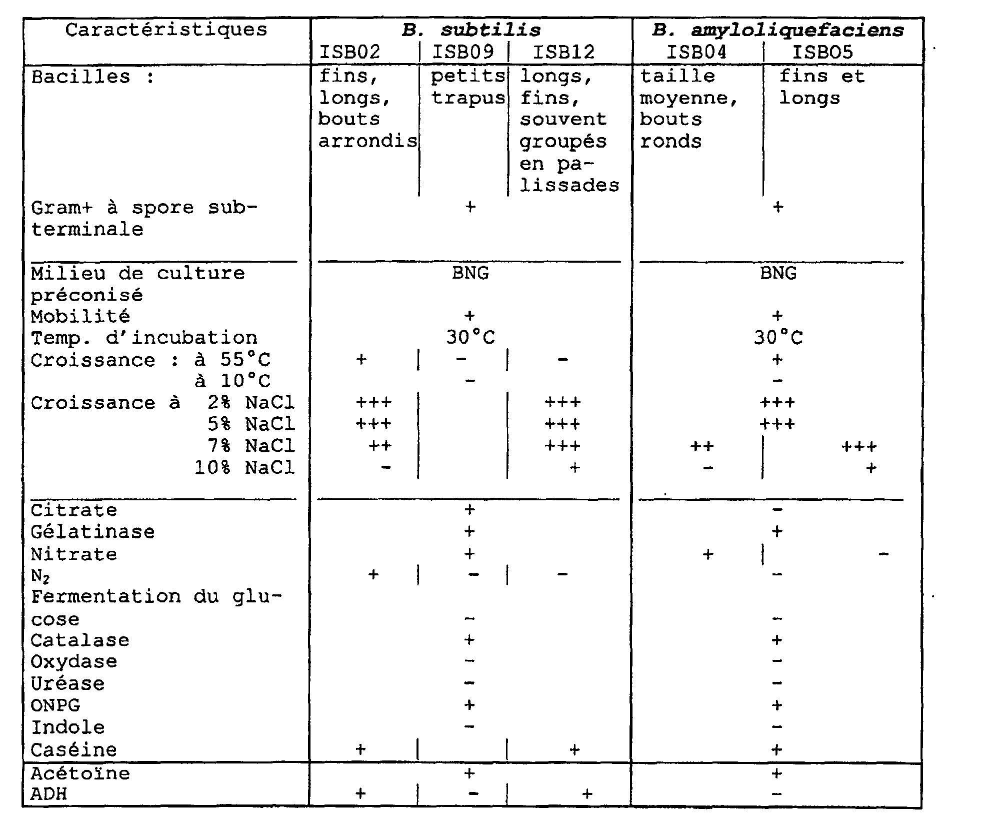

| Caractéristiques | ***B. megaterium*** ISB 06 | ***B. licheniformis*** ISB 07 | ***B. circulans*** ISB 11 |
|---|---|---|---|
| Bacilles : | moyens, à bouts ronds | moyens | moyens, bouts ronds, assez trapus |
| Gram+ à spore sub-terminale | + | + | + |
| Milieu de culture préconisé | BNG | BNG | BNG |
| Mobilité | + | + | + |
| Temp. d'incubation | 30°C | 30°C | 30°C |
| Croissance : à 55°C | + | - | - |
| à 10°C | - | - | + |
| Croissance à 2% NaCl | +++ | +++ | ++ |
| 5% NaCl | +++ | +++ | + |
| 7% NaCl | +++ | ++ | - |
| 10% NaCl | + | - | - |
| Citrate | + | - | - |
| Gélatinase | + | + | + |
| Nitrate | + | + | - |
| N₂ | - | - | - |
| Fermentation du glucose | - | + | - |
| Catalase | + | + | + |
| Oxydase | - | - | - |
| Uréase | - | - | - |
| ONPG | + | + | - |
| Indole | - | - | - |
| Caséine | + | + | - |
| Acétoïne | - | + | - |
| ADH | - | + | - |

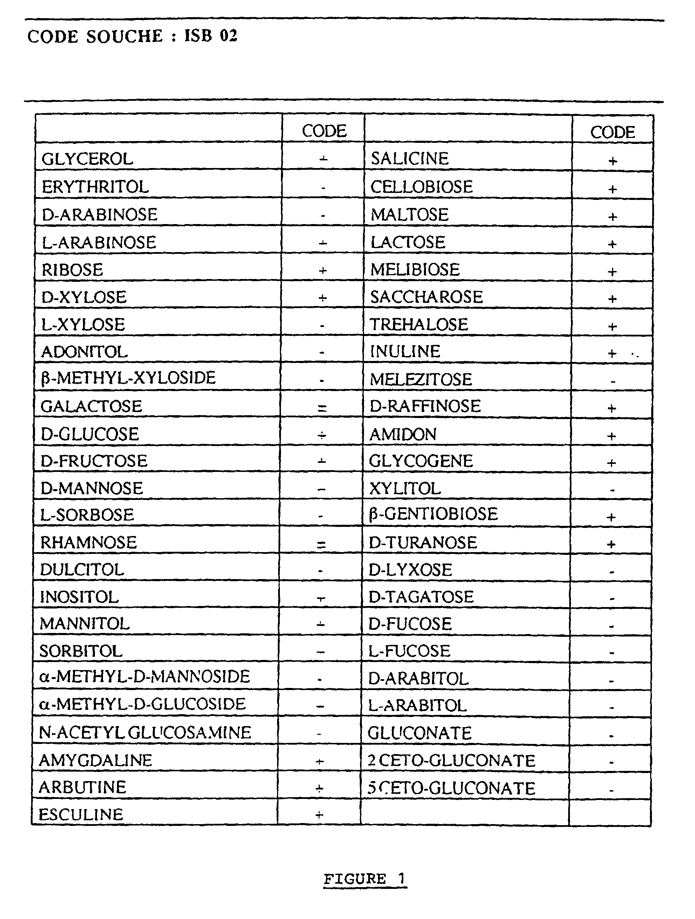

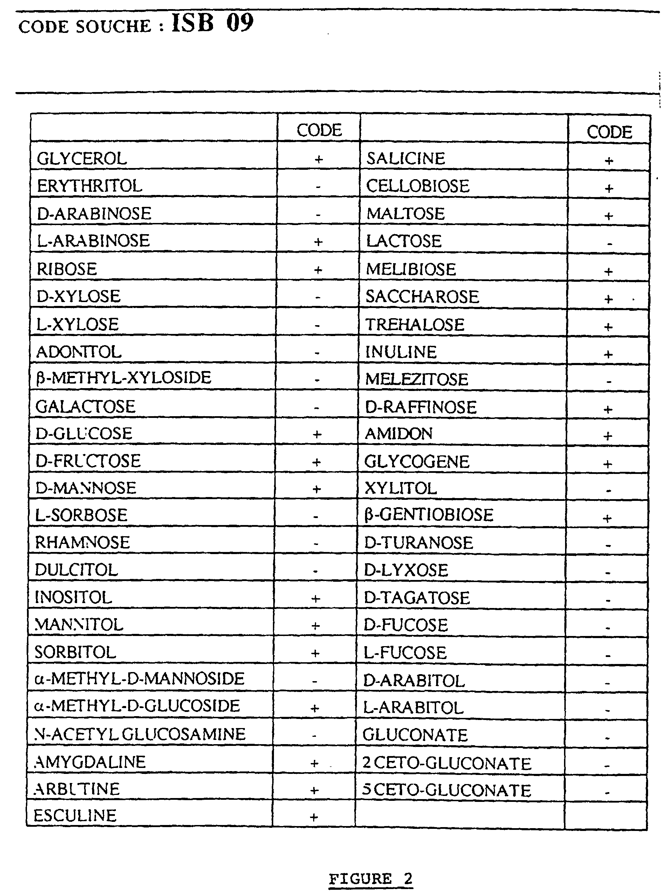

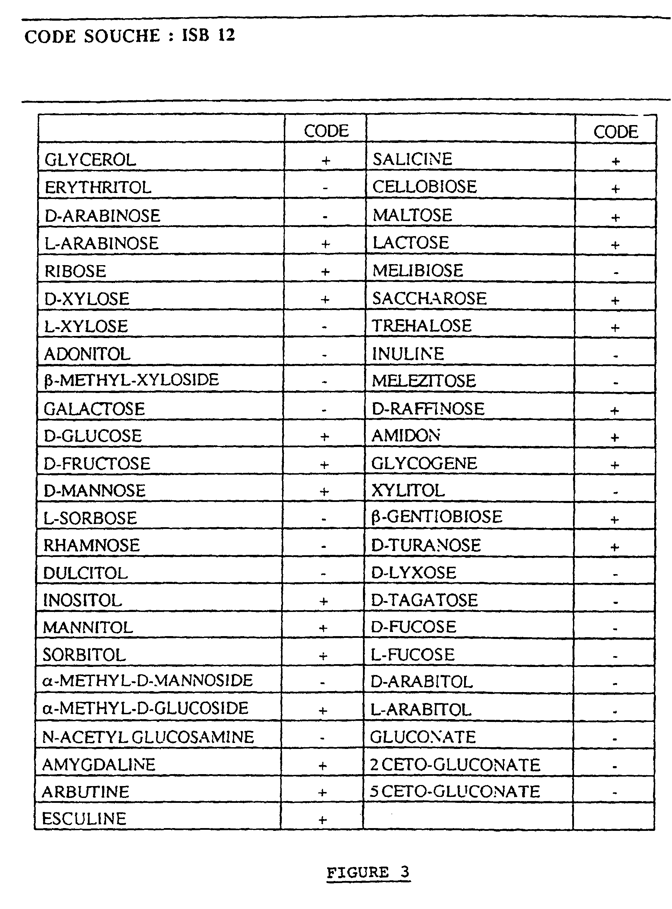

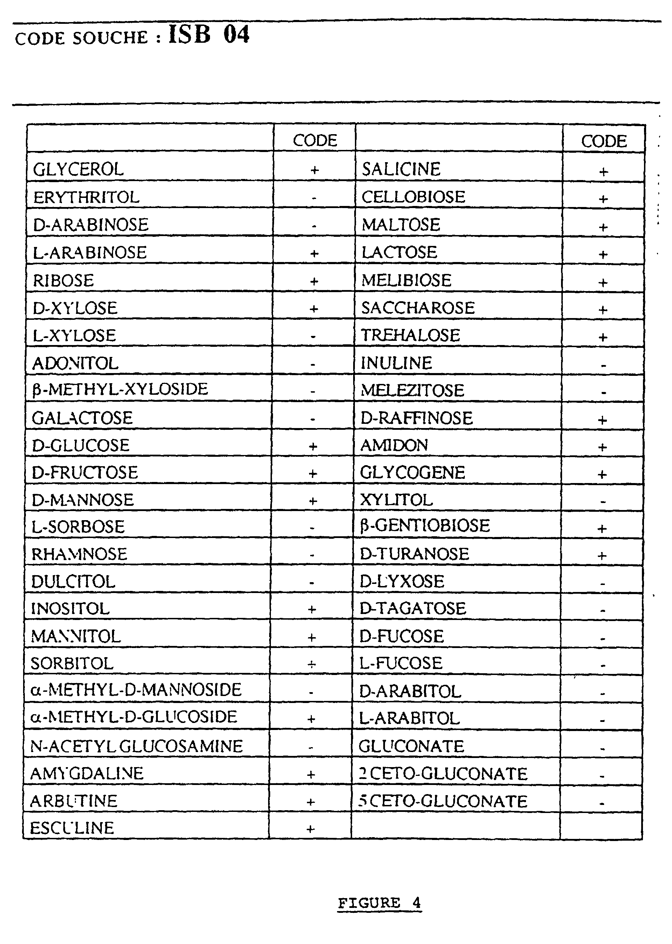

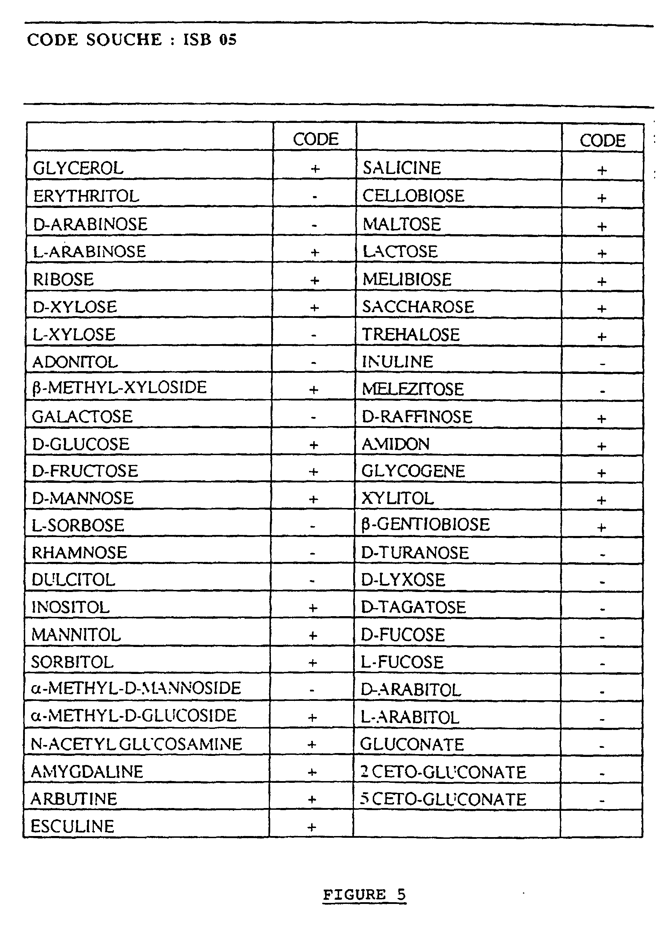

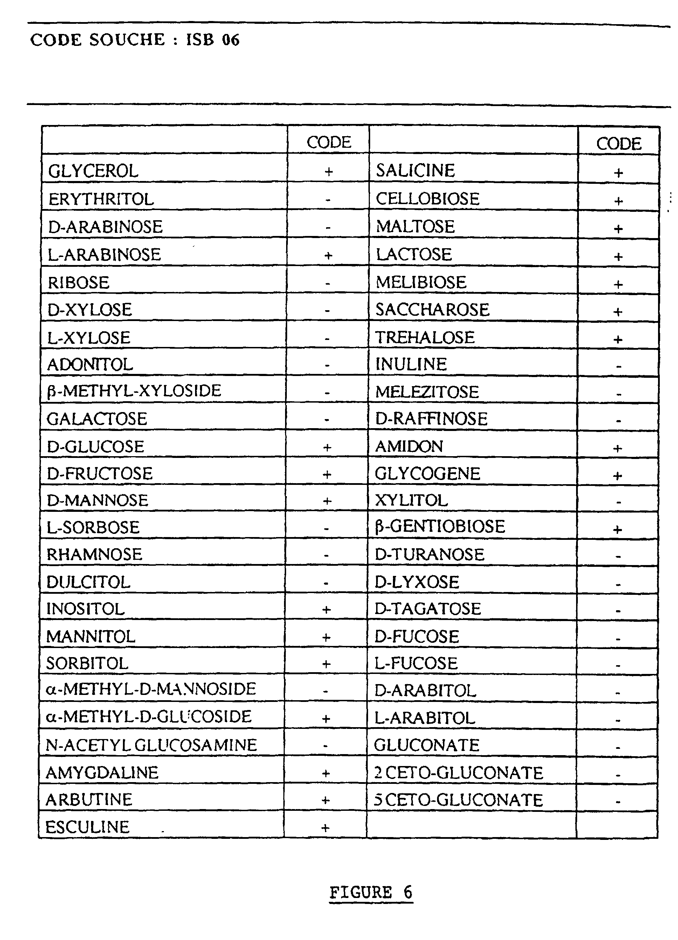

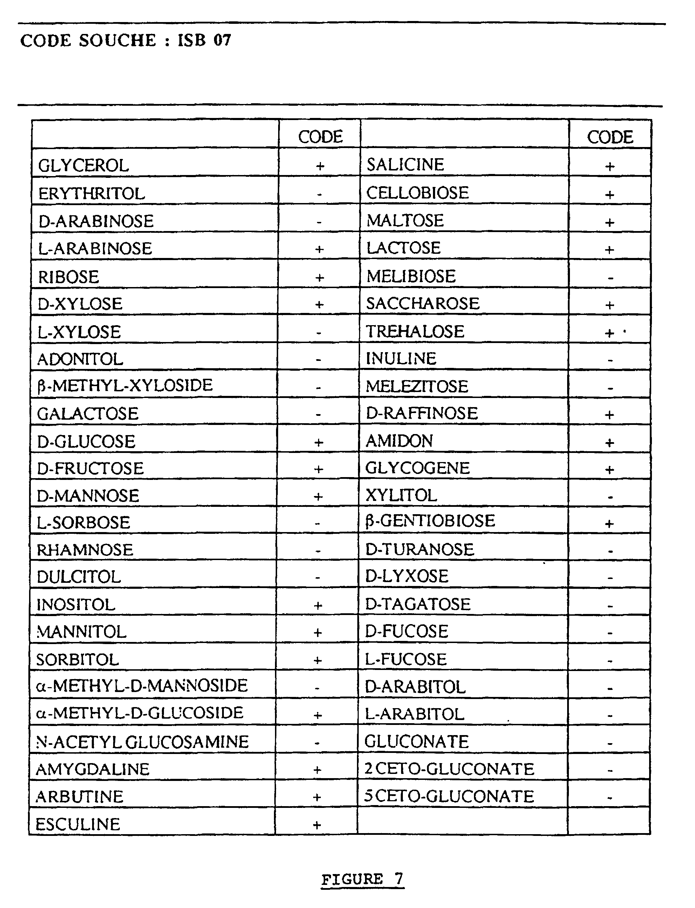

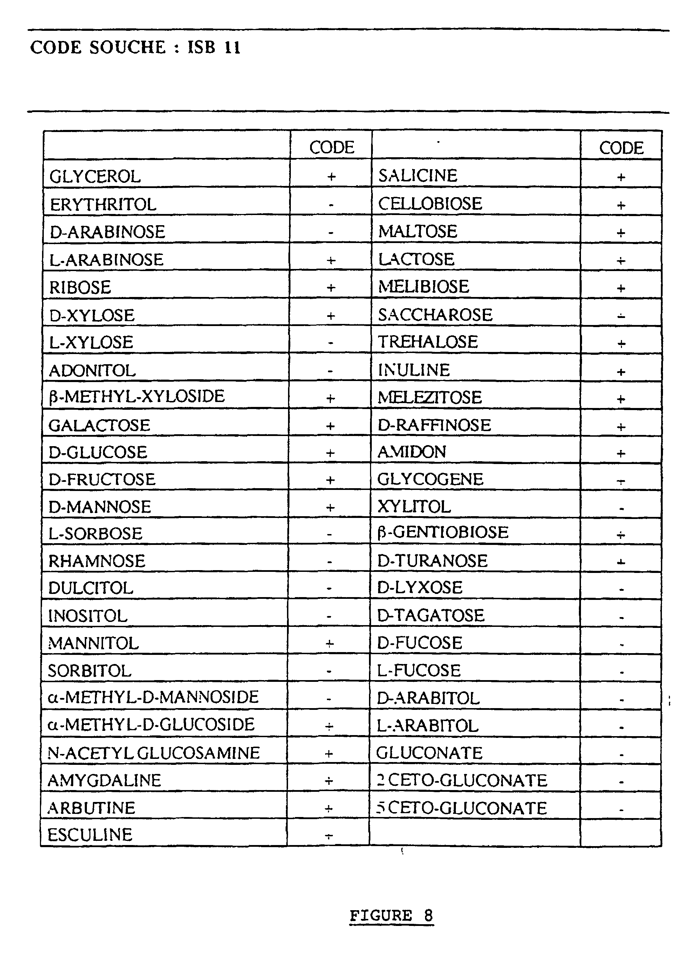

Les résultats des galeries API 50 CH donnant le profil de fermentation des sucres (caractères positifs après 48 h maximum d'incubation) (inoculation selon API) (point 2 dans l'échelle de MacFarland) sont illustrés aux figures 1 à 8.

### EXEMPLE 2 : Caractéristiques des Lactobacillus conformes à l'invention.

Lesdites souches présentent les caractéristiques morphologiques et biochimiques illustrées aux Tableaux suivants :

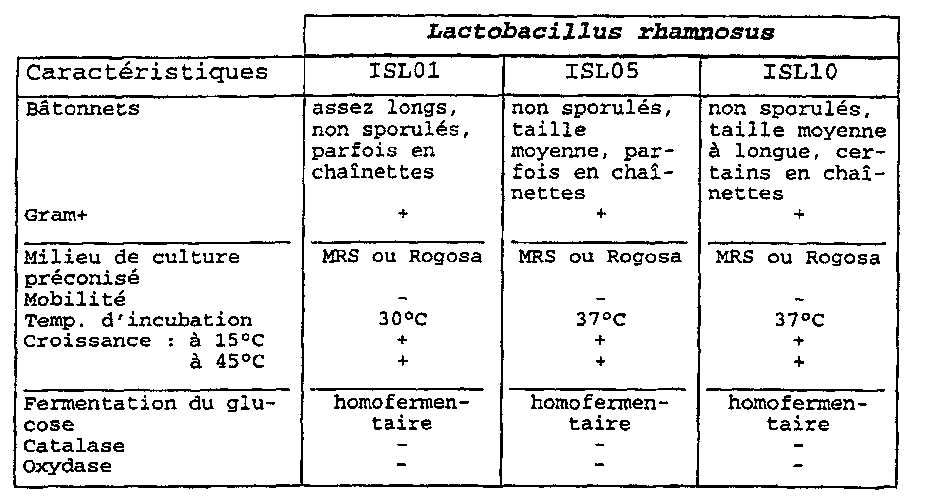

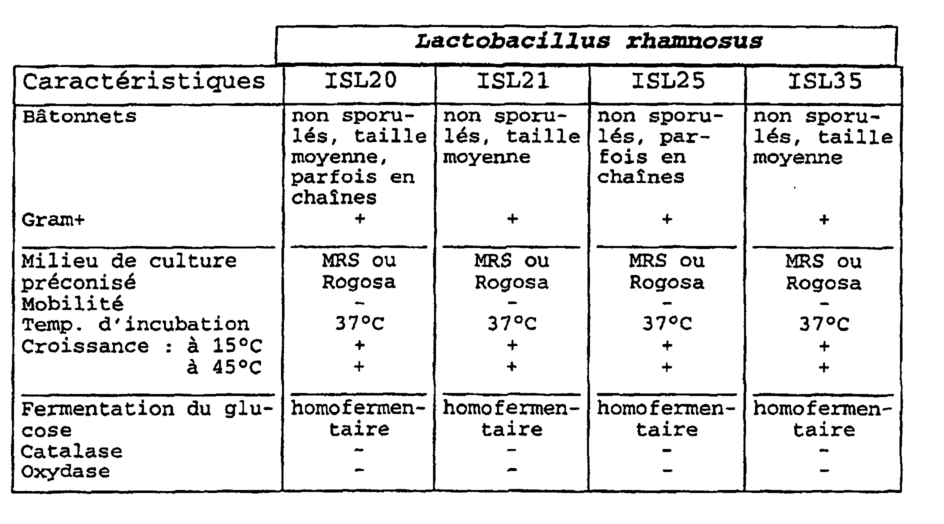

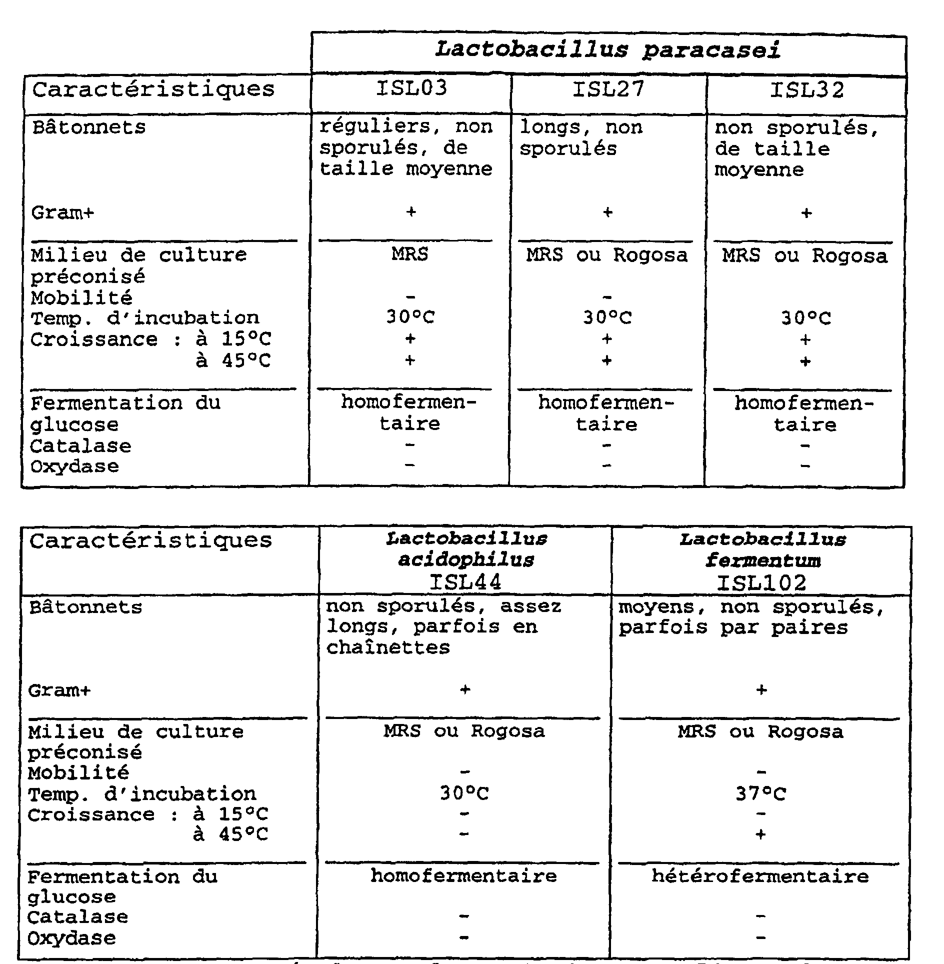

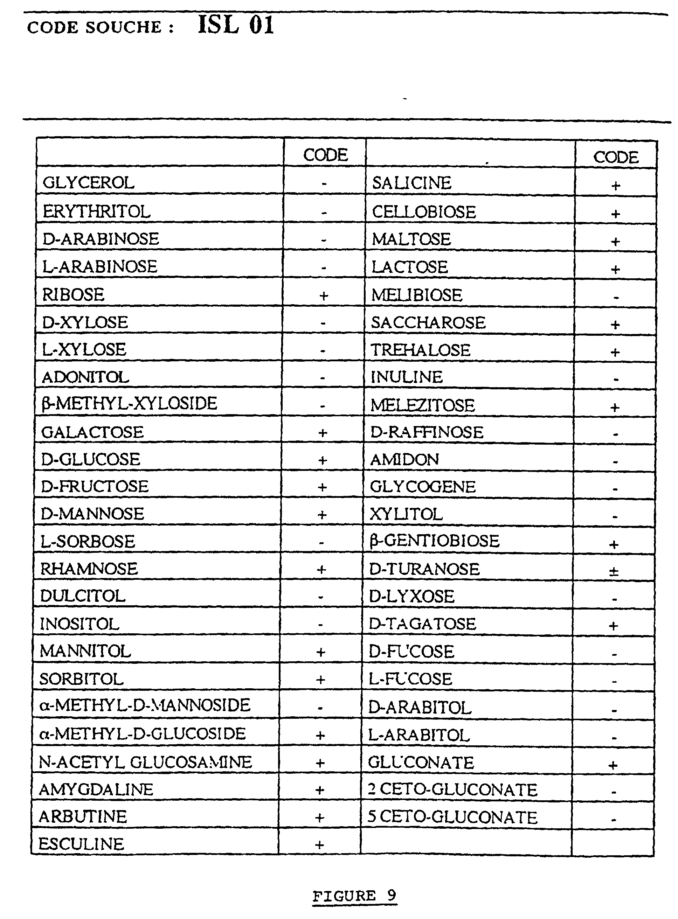

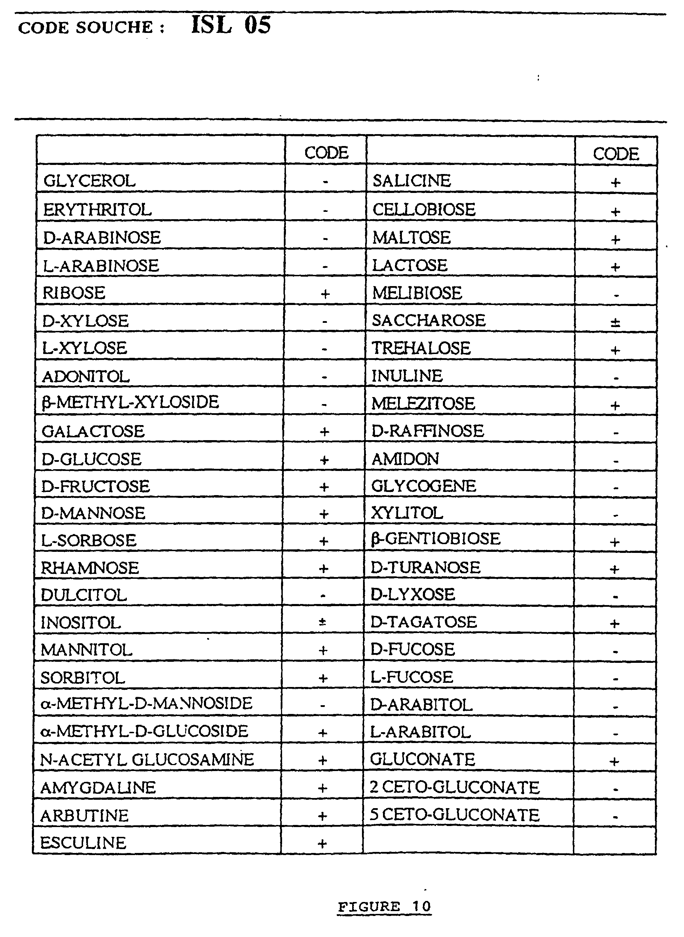

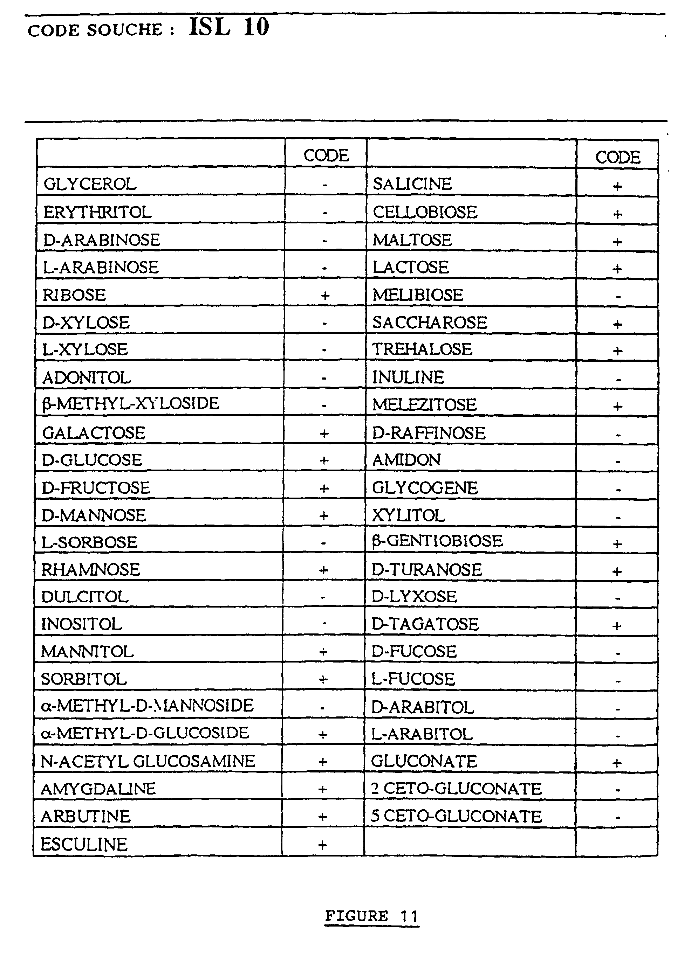

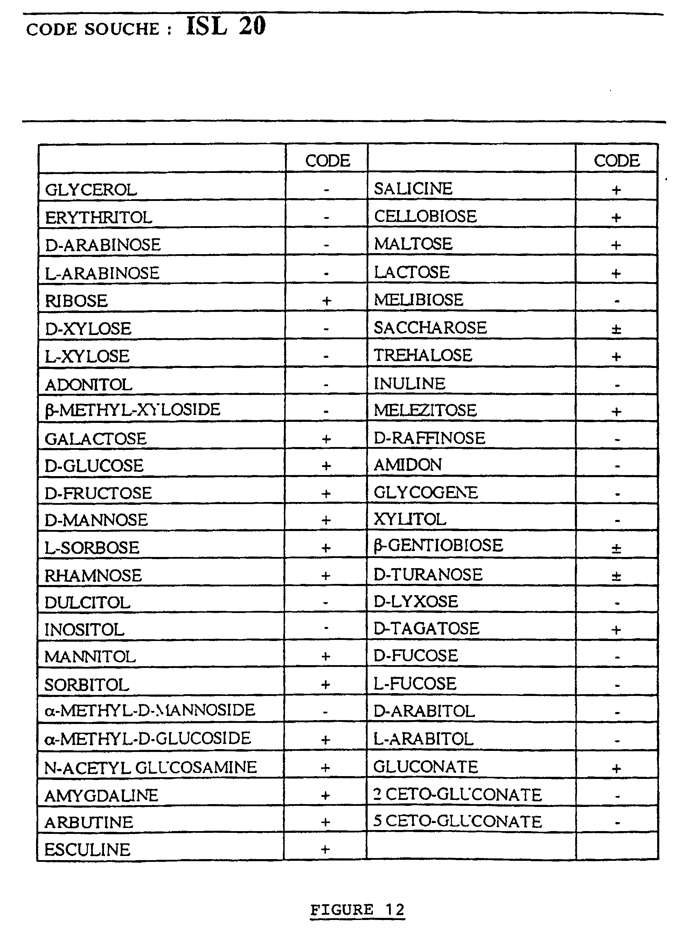

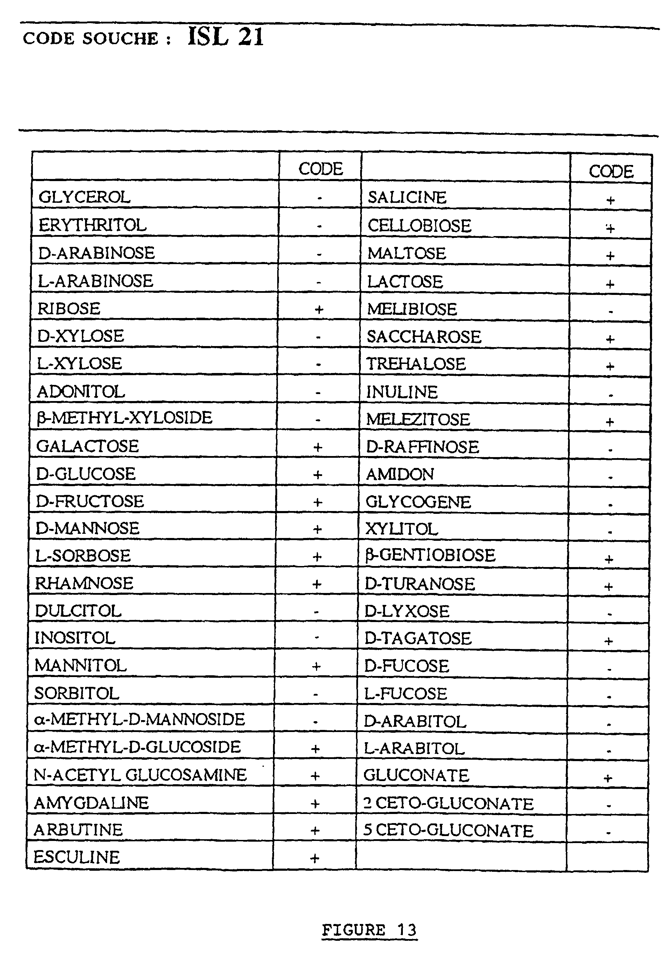

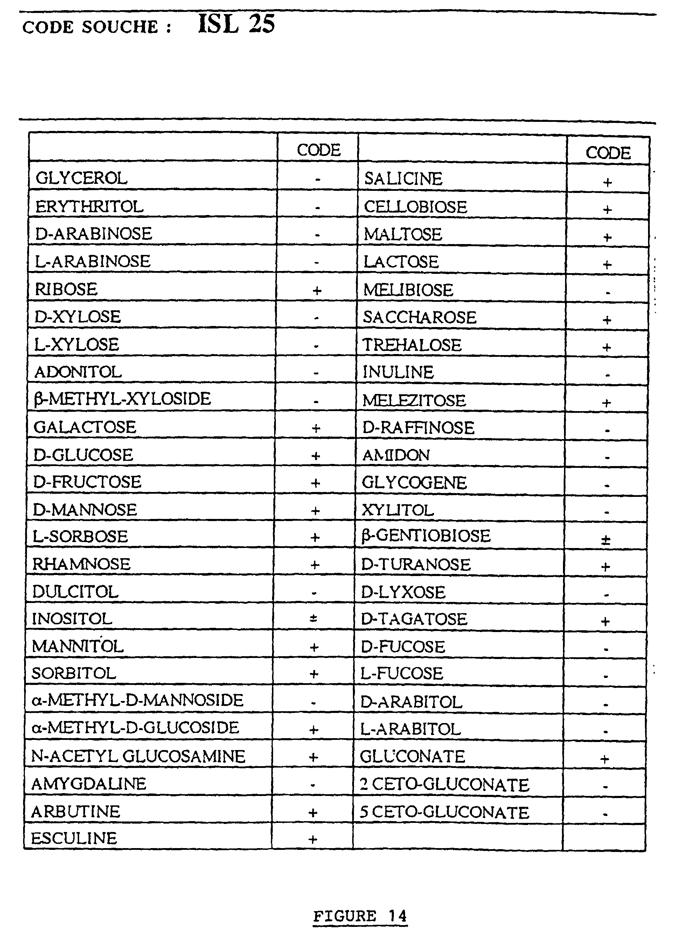

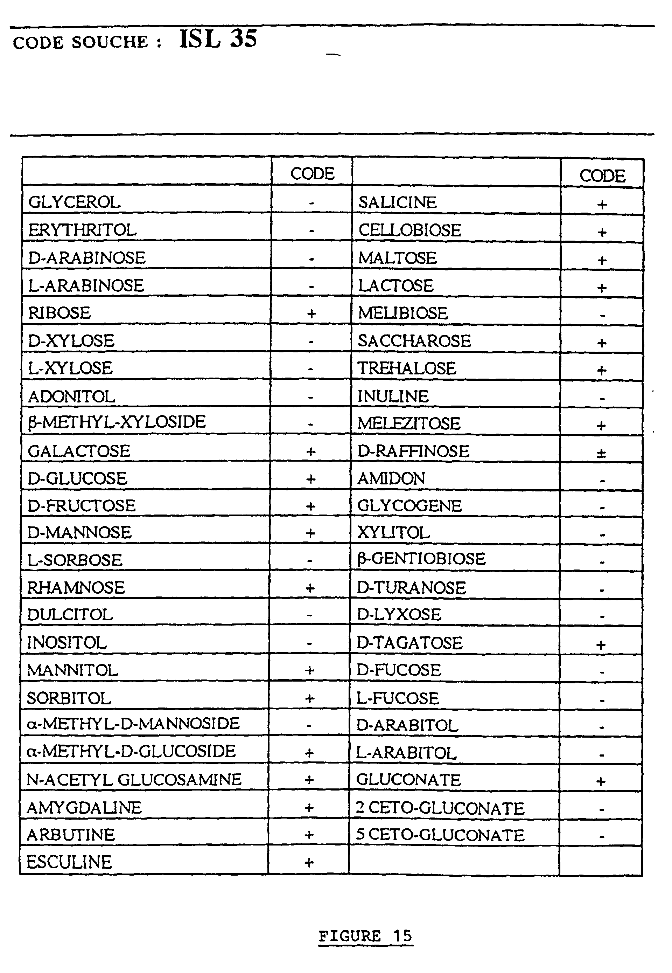

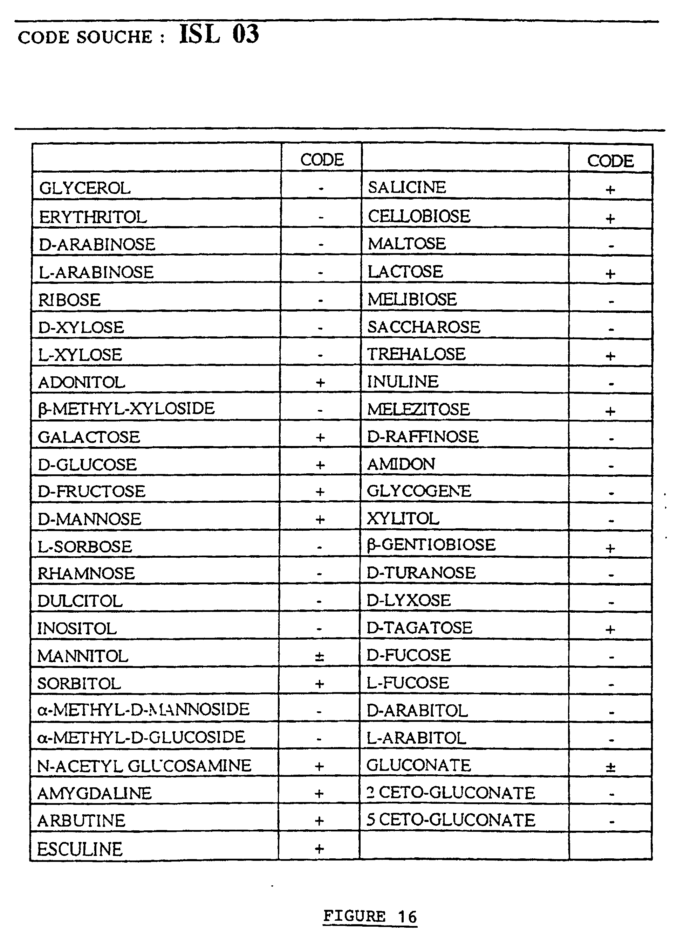

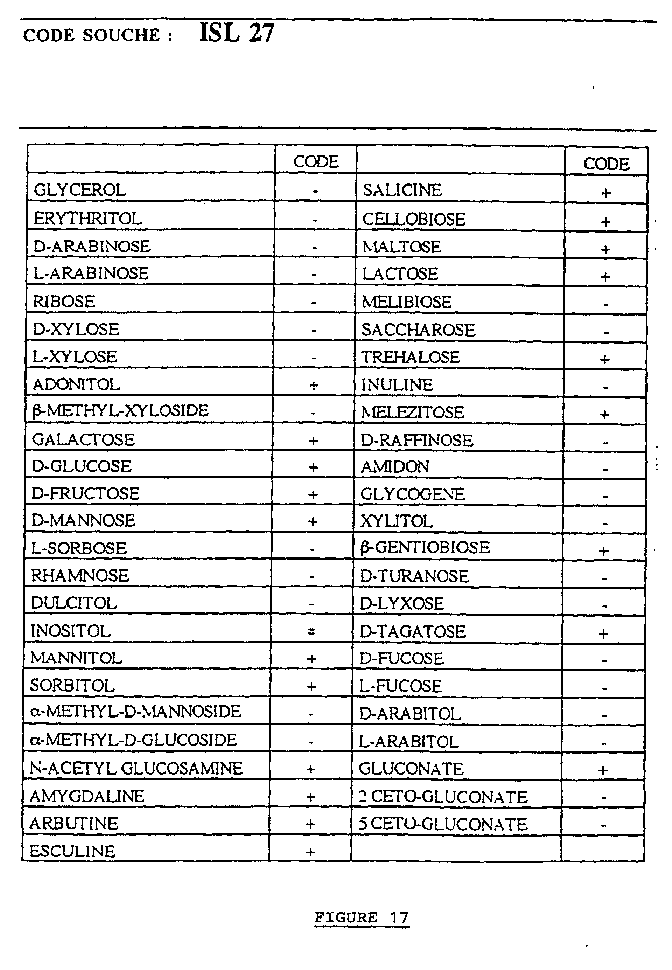

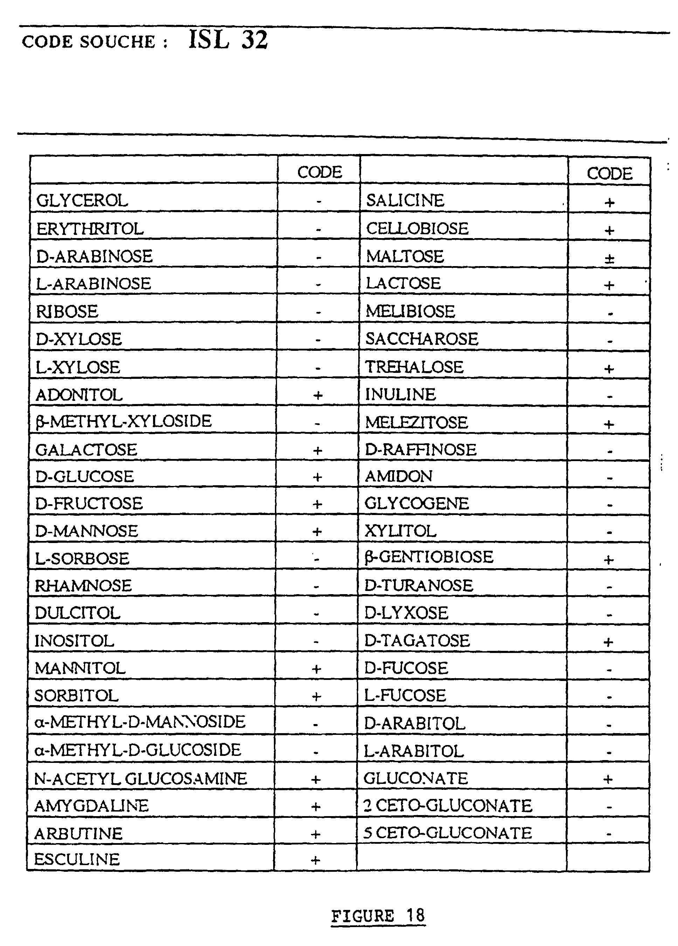

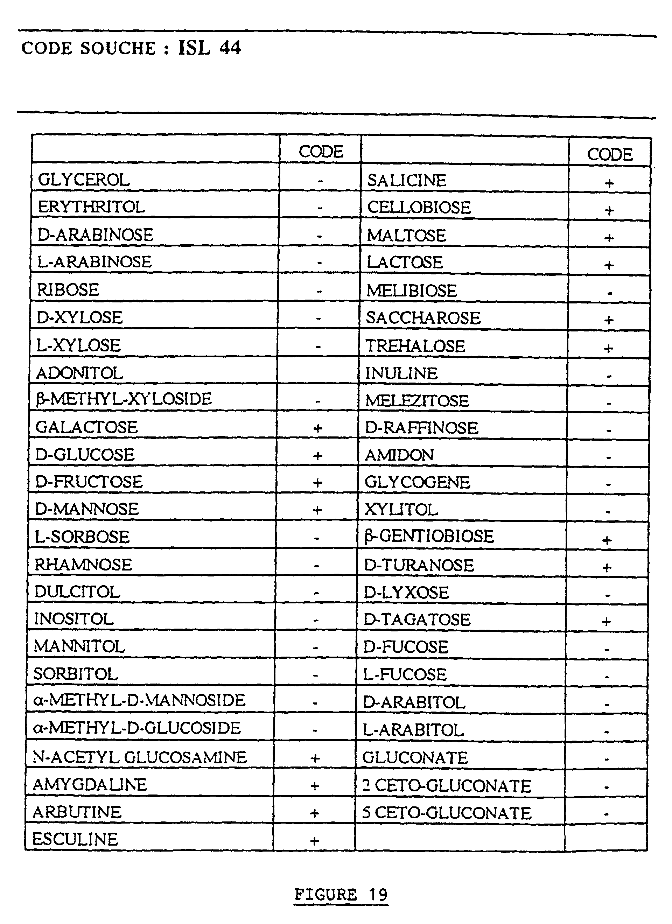

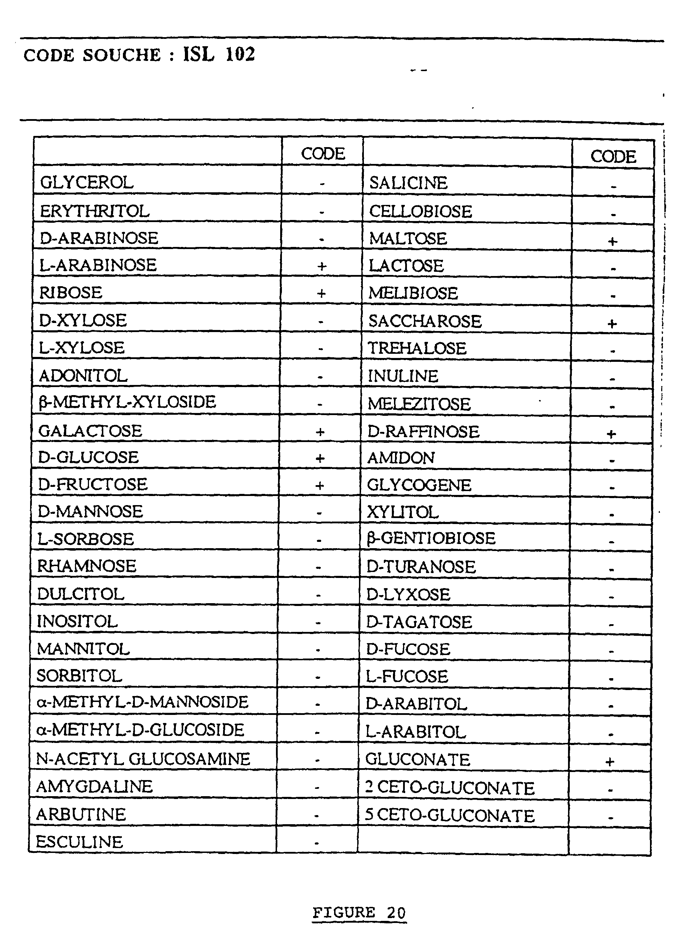

Les résultats des galeries API 50 CH donnant le profil de fermentation des sucres (caractères positifs après 48 h maximum d'incubation) (inoculation selon API) sont illustrés aux figures 9 à 20.

### Exemple 3 : Caractéristiques des Pediococcus conformes à l'invention.

La souche de *Pediococcus pentosaceus* présente les caractéristiques morphologiques et biochimiques illustrées au Tableau suivant :

| Caractéristiques | ***Pediococcus pentosaceus*** ICP01 |
|---|---|
| *Cocci* | en paires ou plus rarement en tétrades ; jamais en chaînettes |
| Gram+ | + |
| Milieu de culture | MRS |
| préconisé | |
| Temp. d'incubation | 37°C |
| Croissance : à 15°C | - |
| à 45°C | + |
| à pH 8 | + |
| à 4% NaCl | + |
| à 6,5%NaCl | + |
| à 15% NaCl | - |
| Fermentation du | homofermentaire |
| glucose | |
| Catalase | - |
| Oxydase | - |
| Arginine | + |
| Nitrate | - |
| ONPG | - |
| Indole | - |
| Acétoïne | - |

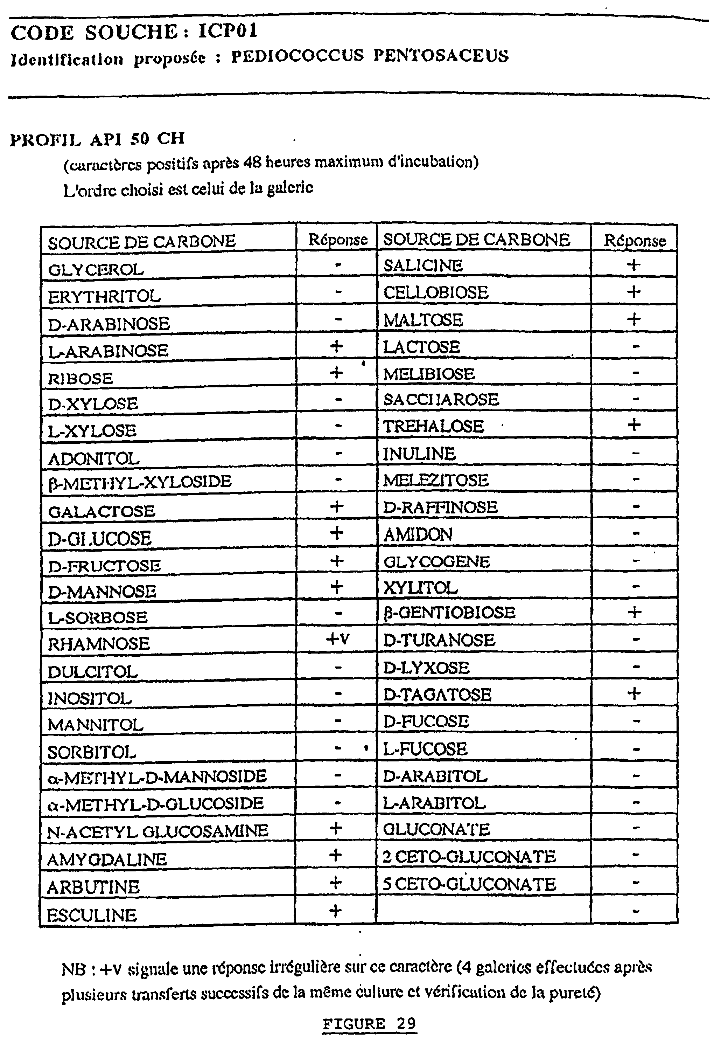

Les résultats des galeries API 50 CH donnant le profil de fermentation des sucres (caractères positifs après 48 heures maximum d'incubation) (inoculation selon API) sont illustrés à la figure 29.

### EXEMPLE 4 : Activités des Bacillus et Lactobacillus.

La sélection de souches présentant une activité enzymatique permet en particulier une digestion contrôlée des déjections.

### 1) Activités enzymatiques :

### * Activité protéolytique :

Pour la majorité des tests, les activités enzymatiques sont mises en évidence sur le milieu minimum de base (MMB), à base d'agar, pour les souches de *Bacil* *lus* et sur le milieu Rogosa, pour les souches de *Lactobacillus.*

Les souches sont ensemencées en stries sur le milieu MMB ou le milieu Rogosa, tous deux additionnés de 1 % (poids/volume) de lait écrémé en poudre, riche en caséine susceptible d'être hydrolysée.

Après une incubation de 24 heures à l'étuve à 30°C pour les *Bacillus* et de 5 jours en étuve anaérobie, à 37°C pour la flore lactique, la présence d'une activité protéolytique se traduit par une clarification du milieu, due à l'hydrolyse de la caséine.

### * Activité amylolytique :

Comme précédemment, les souches bactériennes sont ensemencées en stries sur les deux milieux additionnés de 1 % (poids/volume) d'amidon de blé insoluble. Après incubation dans les mêmes conditions que ci-dessus, l'activité amylolytique se traduit par une clarification du milieu autour des colonies disposées en stries, due à l'hydrolyse de l'amidon.

### * Activité cellulolytique :

Le protocole est le même que pour la recherche de l'activité amylolytique. Le substrat carboné utilisé est la carboxyméthylcellulose.

### * Hydrolyse de l'acide urique :

Le principe est le même que pour la dégradation de l'amidon, l'acide urique étant additionné à une concentration de 1 % (poids/volume), l'hydrolyse de l'acide urique se traduit par une clarification du milieu.

Le Tableau ci-après illustre les résultats et les produits de réaction obtenus :

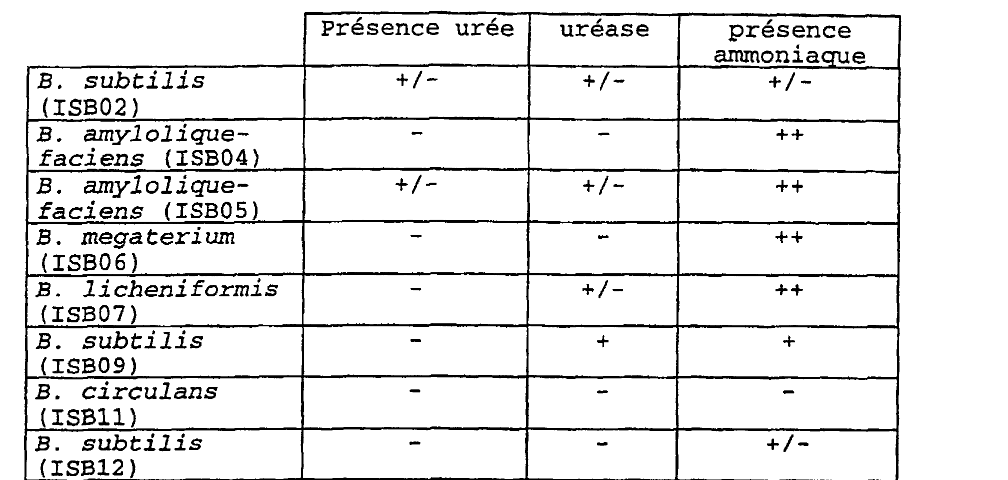

Ces résultats montrent qu'à l'exception de la souche ISB11, l'ensemble des *Bacillus* sont capables de dégrader l'acide urique jusqu'à l'ammoniaque.

### * Hydrolyse de l'urée :

L'hydrolyse de l'urée se visualise en utilisant le milieu de Stuart. Ce milieu contient un indicateur coloré : le rouge de phénol qui vire du jaune au rose foncé dans tout le tube, lorsque l'urée est hydrolysée.

### * Activité lipolytique :

Les différentes souches sont ensemencées sur un milieu tributyrine agar. Avant utilisation les milieux, répartis en tubes, sont agités afin de mettre en émulsion la tributyrine, puis coulés en boîte de Pétri. Après incubation dans les conditions précisées ci-dessus, l'hydrolyse de la tributyrine se visualise par un éclaircissement du milieu autour de la colonie.

Le Tableau ci-après illustre les différentes activités précisées ci-dessus.

| ***Bacillus*** | | | | | | | | | |
|---|---|---|---|---|---|---|---|---|---|
| Code | NO₃ | NO₂ | Urée | Act. Amylolitique | Act. Protéolytique | Act. Lipolytique | 55°C | 60°C | Anaérobie/aérobie facultative |
| ISB02 | + | - | + | + | + | + | + | + | + |
| ISB04 | + | - | - | + | + | + | - | - | + |
| ISB05 | + | - | - | + | + | + | - | - | + |
| ISB06 | + | - | - | + | + | + | - | - | + |
| ISB07 | + | - | - | + | + | + | - | - | + |
| ISB09 | + | - | - | + | + | + | - | - | + |
| ISB11 | | | - | + | +/- | +/- | | | - |
| ISB12 | | | - | + | + | + | | | - |

Les différents bacilles présentent en outre d'autres activités enzymatiques répertoriées dans le Tableau ci-après :

| Activités enzymatiques | Xylanase | CMCase | Cellulase | Kératinase | Pectinase |
|---|---|---|---|---|---|
| *B. subtilis* (ISB02) | + | + | - | ++ | + |
| *B.amylolique-faciens* (ISB04) | + | + | +/- | + | + |
| *B.amylolique-faciens* (ISB05) | + | + | - | - | ++ |
| *B. megaterium* (ISB06) | + | + | +/- | - | + |
| *B. licheniformis* (ISB07) | + | + | - | - | +/- |
| *B. subtilis* (ISB09) | + | + | +/- | - | + |
| *B. circulans* (ISB11) | + | + | ++ | - | - |
| *B. subtilis* (ISB12) | + | + | +/- | - | - |

Les *Lactobacillus* sélectionnés ne présentent pas d'activité amylolytique, protéolytique ou lipolytique.

### 2) Activité du type bactériocine et bactériocine-like :

La recherche de substances inhibitrices est effectuée pour toutes les souches sélectionnées.

Les bactériocines sont des protéines d'origine plasmidique, dont la particularité est d'avoir une activité bactéricide, dirigée contre les bactéries de la même espèce ou d'espèces homologues. Les souches productrices de bactériocines possèdent un gène d'immunité contre leur propre bactériocine. On parle d'activité bactériocine-like pour les activités bactéricides dirigées contre les bactéries hétérologues.

La sélection de souches capables de produire des substances de type bactériocine, inhibitrices est réalisée par l'observation sur boîte de Pétri, d'un éclaircissement autour d'une colonie bactérienne. Cet éclaircissement correspond à l'inhibition des bactéries pathogènes présentes dans la gélose, par les souches sélectionnées.

Les bactéries pathogènes utilisées pour cette recherche sont :
- *Escherichia coli* (sérotype O78K82)
- *Salmonella enteritidis*
- *Salmonella typhimurima*
- *Staphylococcus aureus*
- *Clostridium perfringens*
- *Clostridium septicum*
- *Listeria monocytogenes.*

Il s'agit des principaux pathogènes rencontrés dans les litières contaminées.

Les milieux utilisés pour la culture des bactéries pathogènes sont répertoriés dans le Tableau ci-après.

| Milieux de culture des bactéries pathogènes | | |
|---|---|---|
| Souches | Milieu liquide | Milieu gélosé |
| *Escherichia coli* | BNL | BNG |
| *Staphylococcus aureus* | BNL | BNG |
| *Salmonella* | BNL | BNG |
| *Clostridium* | Bouillon RCM | Gélose RCM |
| *Listeria* | Tryptose | Tryptose gélosé |

Les *Lactobacillus* sont producteurs d'acide lactique, qui est la principale source d'inhibition *in vitro* des bactéries pathogènes.

Pour neutraliser ce facteur, du milieu Rogosa tamponné à pH 6,1, avec un tampon phosphate est utilisé pour la culture des *Lactobacillus.*

Après ensemencement des différentes souches de *Lactobacillus* et incubation, 8h à 37°C, en anaérobiose, chaque colonie est recouverte avec une goutte de milieu spécifique de la bactérie pathogène à tester.

20 ml de milieu gélosé des souches pathogènes spécifiques additionnés de 5 ml d'une culture bactérienne pathogène, sont coulés dans la boîte.

La lecture des boîtes de Pétri s'effectue après 24h d'incubation à 37°C.

La taille des zones d'inhibition produite est mesurée ; le diamètre d'inhibition calculé correspond à la différence entre le diamètre de la zone d'inhibition et le diamètre de la colonie de *Lactobacillus.* Les résultats sont illustrés au Tableau ci-après.

| ***Lactobacillus*** | | | | | | |
|---|---|---|---|---|---|---|
| Code | *S.enteritidis* | *S.typhimurium* | *E. coli* K82 | *Listeria ½* a | *Listeria ½* b | *Listeria ½* c |
| ISL01 | 9 | 6 | 8 | 9 | 11 | 8 |
| ISL03 | 15 | 8 | 9 | - | - | - |
| ISL05 | 6 | 4 | 2 | - | 15 | 15 |
| ISL10 | 7 | 8 | 8 | - | 13 | 11 |
| ISL20 | - | - | 2 | 8 | 23 | 15 |
| ISL21 | - | - | 18 | - | 9 | - |
| ISL25 | 7 | 8 | - | - | 16 | - |
| ISL27 | 10 | 12 | 12 | - | - | - |
| ISL32 | 8 | - | 8 | - | 18 | 15 |
| ISL35 | 10 | 5 | 5 | - | 12 | 22 |
| ISL44 | 12 | 5 | 6 | 7 | 6 | 8 |
| ISL102 | 8 | 0 | 0 | 8 | 0 | 10 |

Pour l'étude de l'action bactériocine des *Bacillus*, les conditions sont les suivantes :

10 ml de milieu PCA sont coulés dans des boîtes de Pétri ; des cultures de 24 heures des souches de *Bacillus* sélectionnées sont repiqués en spot ; après 24 h d'incubation à 30°C, la gélose est décollée avec une spatule stérile et retournée dans une boîte de Pétri de 9 cm de diamètre ; 20 ml de milieu gélosé spécifique des souches pathogènes additionnées de 5 ml d'une pré-culture de ces mêmes bactéries sont coulés dans la boîte de Pétri.

La lecture des boîtes de Pétri s'effectue après 24 h d'incubation, comme précisé ci-dessus pour les *Lactobacillus.*

Le Tableau ci-après illustre les résultats obtenus.

| ***Bacillus*** | | | | | | |
|---|---|---|---|---|---|---|
| Code | *Clostridium perfringens* ⌀ mm | *Clostridium septicum* ⌀ mm | *E. coli* 078K82 ⌀ mm | *Salmonella enteritidis* ⌀ mm | *Salmonella typhimurium* ⌀ mm | *Listeria* |
| ISB2 | 13 | 0 | 0 | 0 | 0 | 0 |
| ISB4 | 19 | 9 | 2 | 2 | 7 | 0 |
| ISB5 | 20 | 22 | 8 | 5 | 4 | 0 |
| ISB6 | 12 | 8 | 5 | 7 | 5 | 0 |
| ISB7 | 9 | 12 | 4 | 8 | 6 | 0 |
| ISB9 | 18 | 11 | 0 | 0 | 0 | 0 |
| ISB11 | 0 | 0 | 0 | 0 | 0 | 0 |
| ISB12 | 10 | 0 | 0 | 0 | 0 | 14 |

Aucune des souches sélectionnées, ne manifeste une activité inhibitrice vis-à-vis de *Staphylococcus aureus.* Par contre ces différentes souches sélectionnées présentent des spectres d'inhibition particulièrement intéressants ainsi que toutes les activités enzymatiques recherchées.

### 3) CMI de différents facteurs de croissance :

### a) Bacillus :

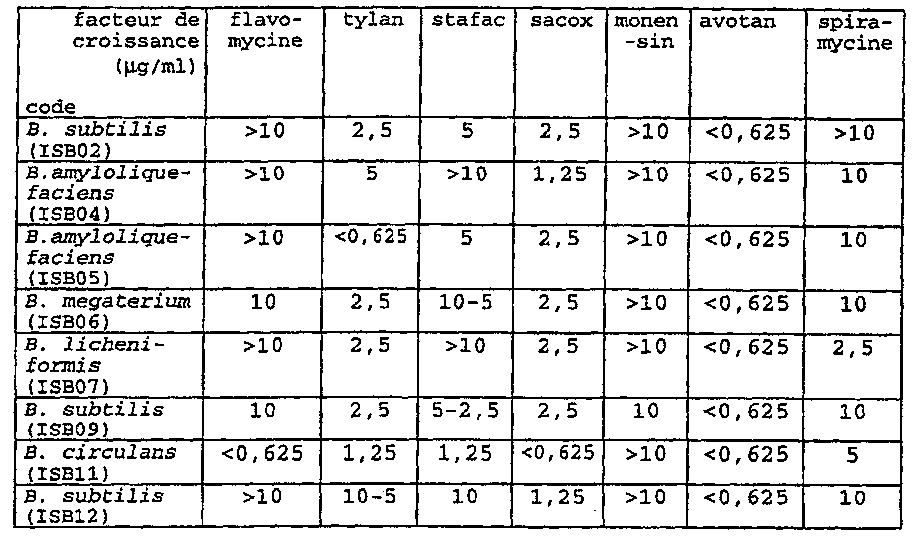

### b) Lactobacillus :

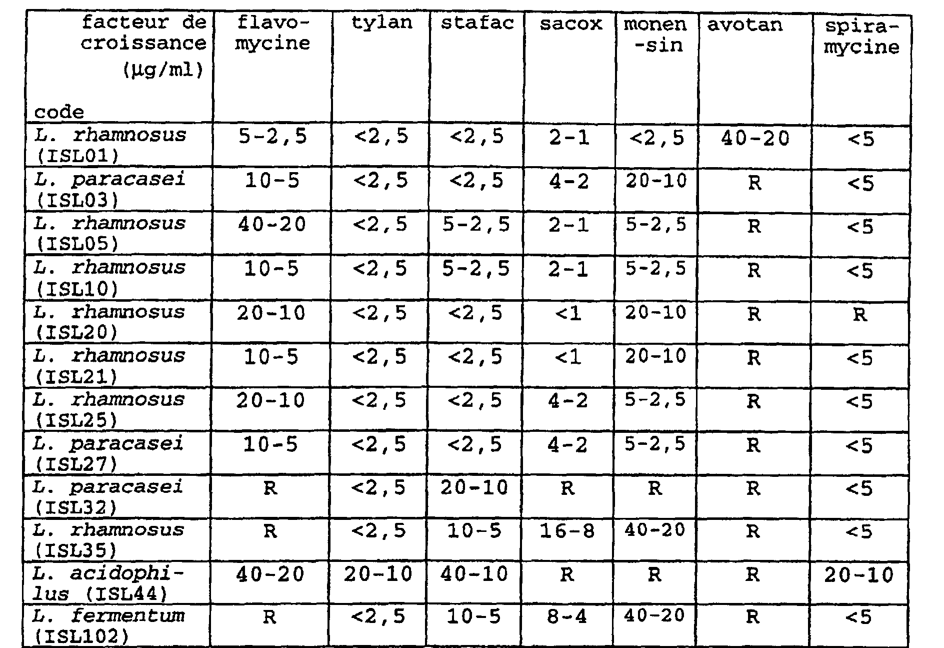

Ces résultats montrent que les souches de *Lactobacillus* testées sont plus résistantes que les souches de *Bacillus ;* on note une forte inhibition des souches de *Bacillus* par l'avotan et le sacox, les CMI respectives étant <0,625 et de 1,25.

La souche de ISL44 est résistante à la majorité des facteurs de croissance.

### Exemple 5 : Activité du type bactériocine et bactériocine-like de Pediococcus pentosaceus.

Après culture de *Pediococcus* sur milieu MRS (de Man, Rogosa et Sharpe) à 4 % de NaCl, on effectue une gamme de dilutions-suspensions jusqu'à 10⁻⁷ ; les dilutions 10⁻¹ à 10⁻⁵ sont ensemencées en surface sur des boîtes de Pétri contenant le milieu MRS, à raison de 0,1 ml par dilution, puis étalées au râteau. Les ensemencements se font en double pour chaque dilution. Les boîtes sont incubées à 37°C pendant 48 h, à l'étuve anaérobie (10 % de CO₂ et 90 % de N₂).

Avant de récupérer le surnageant de culture, on vérifie la pureté de la souche en ensemençant les souches sur des boîtes de MRS gélosé, selon la technique des quadrants (culture 48 h à 37°C à l'étuve anaérobie). Une colonie bien isolée permet d'inoculer un tube de 10 ml de bouillon MRS, qui est ensuite mis à l'étuve 24 h à 37°C, en anaérobiose. 1 ml de chaque préculture est ensemencé dans 50 ml de bouillon MRS et l'incubation est effectuée pendant 16 h dans les mêmes conditions ; 20 ml de surnageant de culture sont prélevés au terme de l'incubation de 16 h et centrifugés à 10 000 g. Le surnageant débarrassé des cellules et contenant les produits excrétés est récupéré.

### * préparation des différents témoins

Plusieurs traitements sont effectués sur le surnageant obtenu, afin de s'assurer que l'inhibition est bien due à la présence d'une bactériocine ; en effet, le surnageant présente un pH acide (environ 4,5), contient des acides organiques produits par ladite souche (acide acétique et acide lactique), du peroxyde d'hydrogène (absence de catalase) :
. inhibition de l'action du pH: neutralisation à pH 6,5-7,5 (test du pH);
. évaluation de l'action des acide organiques : témoin contenant uniquement ces acides (test des acides) ;
. inhibition de l'action du peroxyde d'hydrogène : témoins avec catalase (incubation de 0,5 ml de catalase avec 0,5 ml de surnageant de culture de *Pediococcus pentosaceus* pendant 1 heure à 37°C, puis refroidissement une heure à température ambiante, avant d'effectuer le test d'inhibition) (test de la catalase) ;
. évaluation de l'action de la température (chaleur ou froid) (tests du froid et de la chaleur) ;
. évaluation de l'action des enzymes protéolytiques (trypsine, α-chymotrypsine, pronase E), selon un protocole identique à celui utilisé pour la catalase (tests de la trypsine, de la pronase et de l'α-chymotrypsine) .
. évaluation du poids moléculaire de la bactériocine produite (test de dialyse).

### * test d'inhibition de S. aureus

Tous les échantillons préparés (surnageant de culture et différents témoins) sont stérilisés à l'aide de filtres 0,2 µm ; le test est effectué comme suit :

On coule dans deux boîtes de Pétri 100 ml de BNG, maintenu à 45°C et auquel on a rajouté 0,5 ml d'une culture de 16 h de *S. aureus.* L'ensemble est laissé à solidifier ; à l'aide d'un emporte-pièce, 9 puits sont creusés dans chaque boîte. Les boîtes sont à nouveau laissées à solidifier pendant environ 1 h à 4°C. Chaque puits est alors rempli avec 100 µl d'échantillon (surnageant de culture ou témoin), comme illustré dans le tableau ci-après.

| n° du puits | Boîte de Pétri I | Boîte de Pétri II |
|---|---|---|
| 1 | surnageant témoin | surnageant témoin |
| 2 | test catalase | test du froid |
| 3 | test catalase témoin* | test de la chaleur |
| 4 | test trypsine | test pH |
| 5 | test pronase E | test des acides |
| 6 | test pronase E témoin* | test dialyse |
| 7 | test trypsine témoin* | test tampon seul |
| 8 | test α-chymotrypsine | rien |
| 9 | test α-chymotrypsine témoin* | rien |

| | | |
|---|---|---|
| * sans surnageant de culture | | |

Les boîtes sont placées à 4°C pendant environ 2 heures, afin que la bactériocine diffuse dans la gélose, puis le tout est mis à l'étuve à 37°C pendant 24 h.

### * résultats

Le Tableau ci-après illustre les résultats obtenus.

| n° de puits | Diamètre inhibition Boîte I | Diamètre inhibition Boîte II |
|---|---|---|
| 1 | 13 | 12 |
| 2 | 11 | 11 |
| 3 | 0 | 6 |
| 4 | 12 | 11 |
| 5 | 10 | 0 |
| 6 | 0 | 11 |
| 7 | 0 | 0 |
| 8 | 12 | 0 |
| 9 | 0 | 0 |

Ces résultats montrent que *Pediococcus pentosaceus* produit une bactériocine effectivement active sur *S. aureus.*

### Exemple 6 : Mise en évidence de la synergie d'action entre les Bacillus et les Lactobacillus dans un complexe bactérien selon l'invention.

1) comparaison des temps de génération (Tg), du nombre d'ufc/ml et du taux de survie pour quelques souches de *Lactobacillus* en monoculture/à leur association dans un complexe bactérien selon l'invention (sur un milieu constitué de fientes, stérile et ensemencé avec les bactéries citées) :

| Souches de *Lactobacillus* | Tg (h) | ufc/ml (final) | taux de survie (%) |
|---|---|---|---|
| *L. rhamnosus* (ISL01) | 13 | 3,30.10⁷ | 22,4 |
| *L. rhamnosus* (ISL01) + *B. subtilis* (ISB02) | 6 | 6,40.10⁷ | 52,4 |
| *L. rhanmosus* (ISL01) + *B. megaterium* (ISB06) | 8 | 1,20.10⁷ | 50,0 |
| *L. rhamnosus* (ISL01) + *B. subtilis* (ISB09) | 8 | 5,40.10⁸ | 23,7 |
| *L. rhamnosus* (ISL21) | 9,8 | 1,00.10⁸ | 100 |
| *L. rhamnosus* (ISL21) + *B. circulans* (ISB11) | 4 | 2,01.10⁷ | 79,7 |

2) comparaison du temps de génération et du nombre d'ufc/ml des souches de *Bacillus* en monoculture et en coculture :

| Souches de *Bacillus* | Tg (h) | ufc/ml max. avt sporulation |
|---|---|---|
| *B. megaterium* (ISB06) | 2,9 | 7,3.10⁸ |
| *B. megaterium* (ISB06) *+ L. paracasei* (ISL27) | 0,8 | 1,20.10⁸ |
| *B. circulans* (ISB11) | 3,2 | |
| *B. circulons* (ISB11) + *L. rhamnosus* (ISL21) | 0,28 | 2,40.10⁸ |

3) dégradation d'acide urique

| | 24 h (%) | 48 h (%) |
|---|---|---|
| témoin | 0 | 0 |
| *L. paracasei* (ISL27) | 60 | 71 |
| *B. megaterium* (ISB06) + *L. paracasei* (ISL27) (avec glucose) | 75 | 91 |
| *L. rhamnosus* (ISL21) | 48 | 63 |
| *B. circulans* (ISB11) + *L. rhamnosus* (ISL21) (avec glucose) | 72 | 85 |

### EXEMPLE 7 : Complexe bactérien pour litière de ruminants et de porcs.

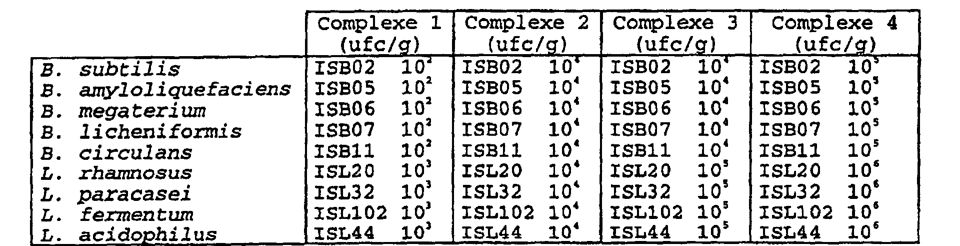

### EXEMPLE 8 : Complexe bactérien pour litières de volailles.

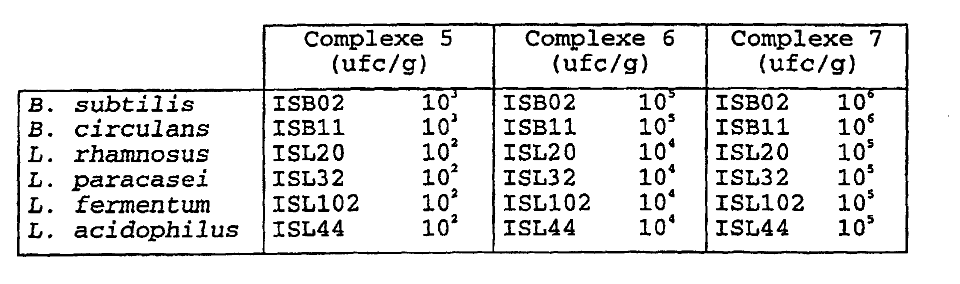

### EXEMPLE 9 : Complexe bactérien pour lisiers et pour fosses septiques.

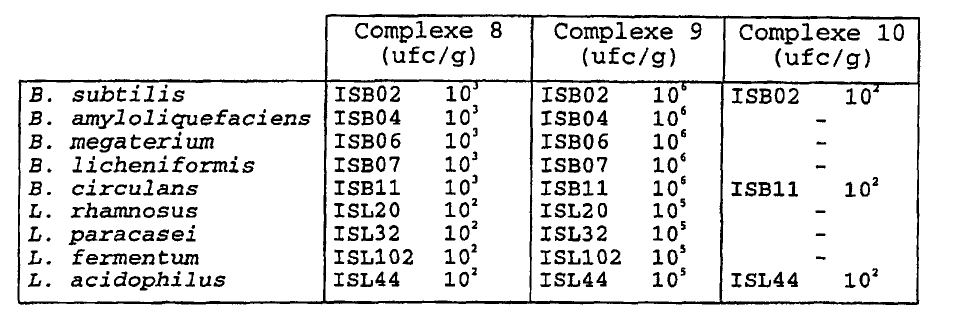

### EXEMPLE 10 : Complexe bactérien pour fumier (compost).

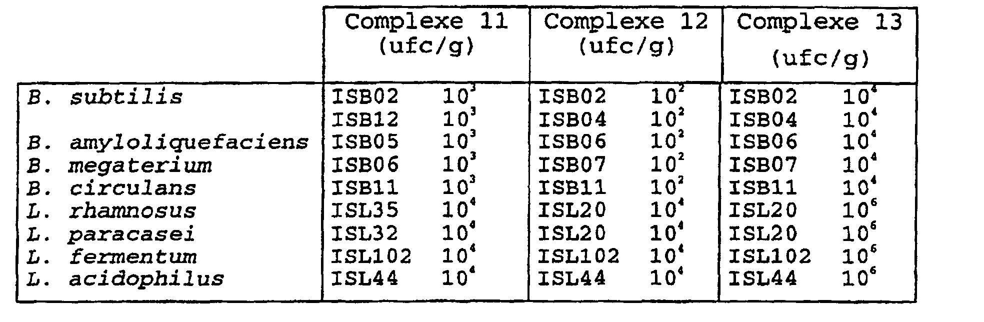

### EXEMPLE 11 : Complexe bactérien pour nettoyage de lagune.

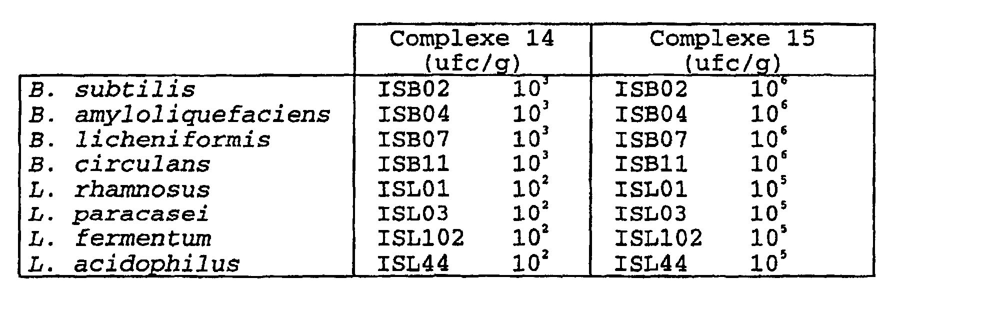

### EXEMPLE 12 : Complexe bactérien pour traitement de cadavres animaux.

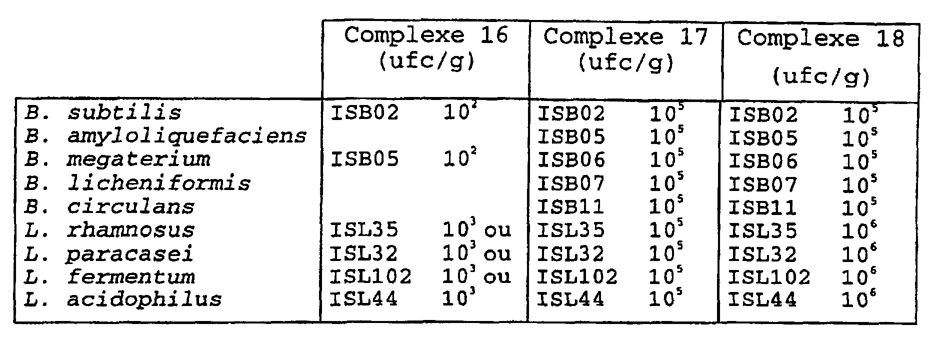

### EXEMPLE 13 : Comparaison litières traitées/litières non traitées.

### a) Ensemencement des litières :

Les litières sont ensemencées de telle sorte que la concentration en *Bacillus* soit de 100 ufc/g de paille (2.10³ ufc de *Bacillus* pour 20 g de produit pour traitement de litière (complexe ou composition de traitement conforme à l'invention)).

Deux méthodes différentes d'ensemencement expérimentales des litières ont été utilisées.

Une première méthode consiste en l'ensemencement de 400 g de litière placés dans une boîte hermétiquement fermée, un complexe bactérien conforme à l'invention. L'incubation se fait à 30°C. Après 3 et 7 jours d'incubation, 20 g de litière sont prélevés. Les échantillons sont ensuite préparés comme suit :

20 g de produit à analyser sont mélangés avec 180 ml de diluant tryptone sel puis homogénéisés au Stomacher (Lab. Blender) pendant 2 min. A partir de cette suspension mère (→dilution au 1/10ème), une gamme de dilutions est réalisée pour faire l'analyse microbienne.

Dans la seconde méthode utilisée, pour améliorer l'homogénéité des ensemencements, 20 g de litière sont déposés dans un sac Stomacher avec filtre, qui évite de prélever des résidus de litière lors des dilutions. L'ensemencement est directement réalisé dans les sacs qui sont incubés suivant le temps désiré à 30°C, à chaque temps correspond un sac. 60 ml de diluant tryptone-sel sont ensuite mélangés à la litière, puis une gamme de dilutions décimales est réalisée.

Les numérations sont effectuées sur :
- la flore thermorésistante (*Bacillus*)
- les coliformes fécaux,
- les anaérobies sulfito-réducteurs (*Clostridium*),
- la flore lactique *(Lactobacillus),*
conformément aux protocoles usuels de culture.

La présence de *Salmonella* est également recherchée.

### b) Evolution de l'azote (solubilité, azote ammoniacal, azote aminé) et de la matière sèche d'une litière traitée avec un complexe selon l'invention (litière bovine, selon l'exemple 4) :

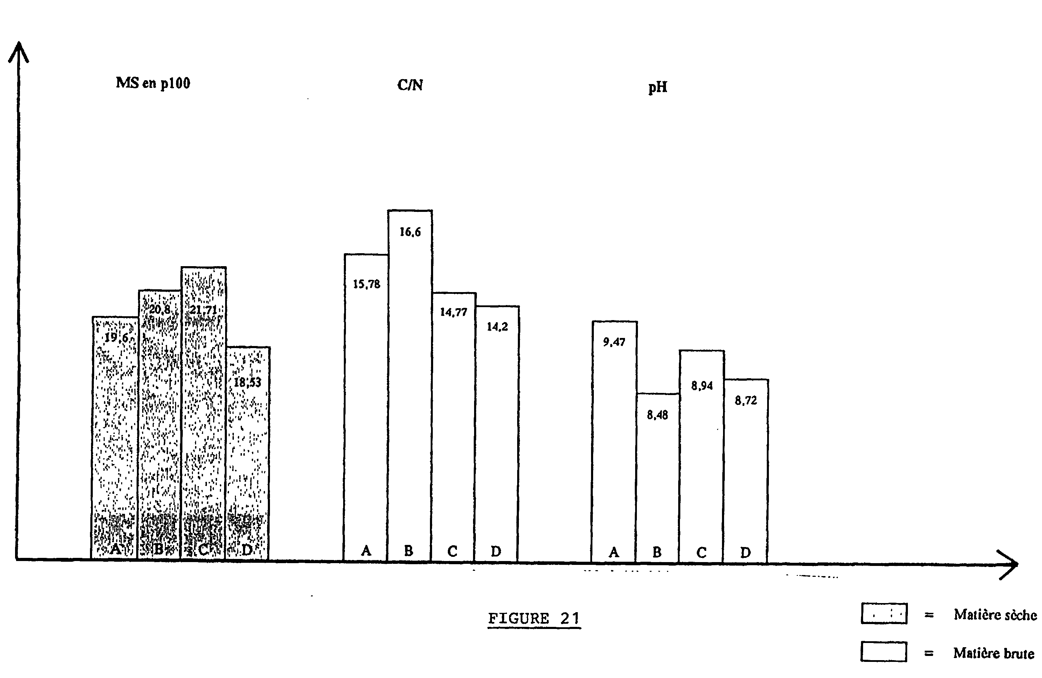

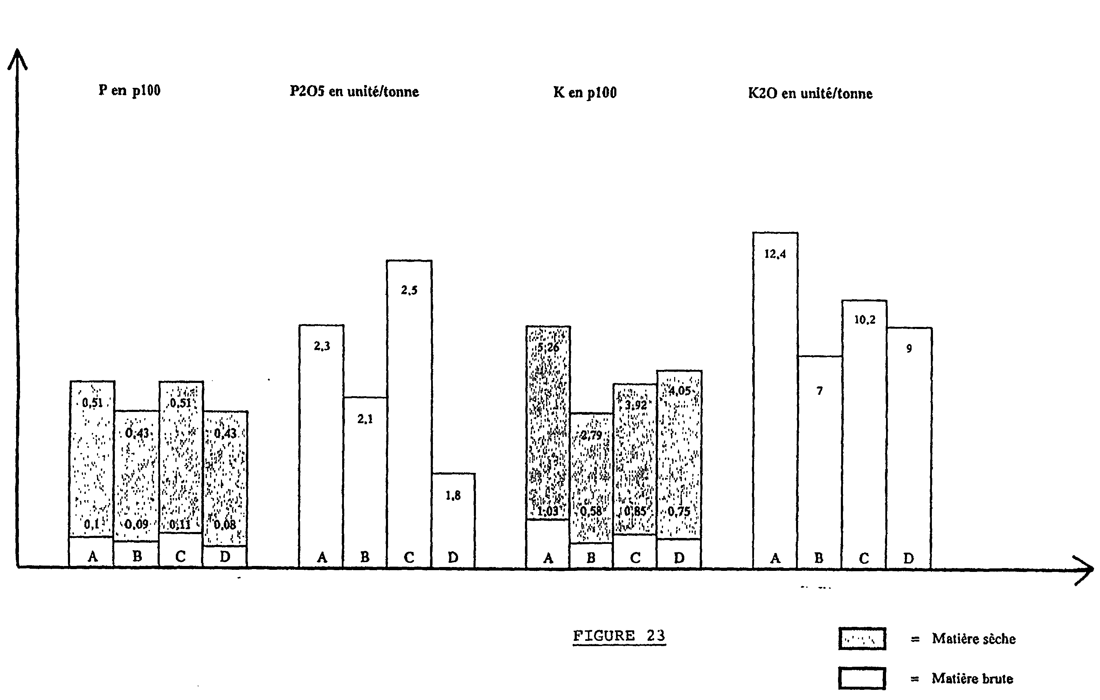

Les figures 21 à 23 montrent l'évolution au cours du temps de la matière sèche (en pourcentage), du rapport C/N, du pH, de l'azote total (en pourcentage), de la solubilité de l'azote (en pourcentage), du pourcentage d'azote ammoniacal par rapport à l'azote total, du pourcentage d'azote aminé, des concentrations en nitrate et des taux en phosphore, phosphates et potassium.

Dans ces figures, les colonnes A représentent les litières non traitées, en place depuis 2 semaines, les colonnes B, C et D représentent les litières traitées avec un complexe bactérien selon l'exemple 4, en place depuis 7 semaines (colonnes B), en place depuis 16 semaines (colonnes C) et en place depuis 12 semaines et stockées depuis 8 semaines (colonnes D).

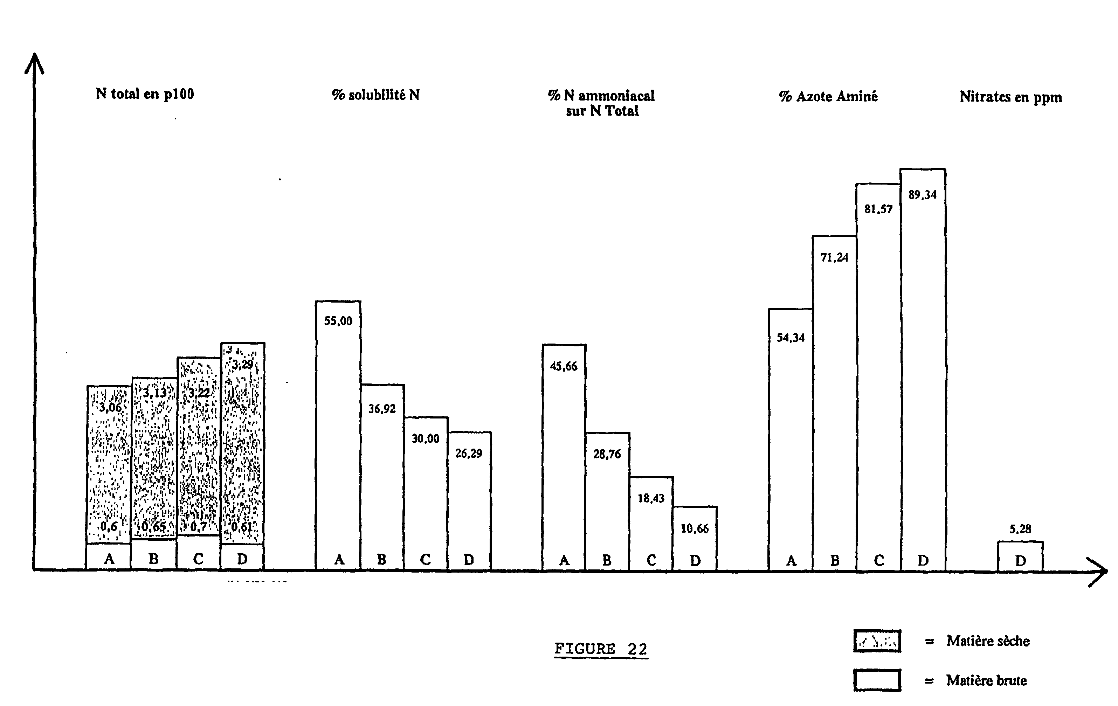

Ces figures montrent qu'après traitement avec un complexe bactérien conforme à l'invention, il y a stabilisation de l'azote, diminution significative de l'azote sous forme soluble et augmentation du pourcentage d'azote aminé (figure 22).

Par ailleurs, alors qu'une putréfaction normale entraîne une hydrolyse et une baisse de la matière sèche, le traitement conforme à l'invention permet d'éviter une telle baisse (figure 21).

### c) Comparaison des teneurs en nitrates de fumiers différemment traités :

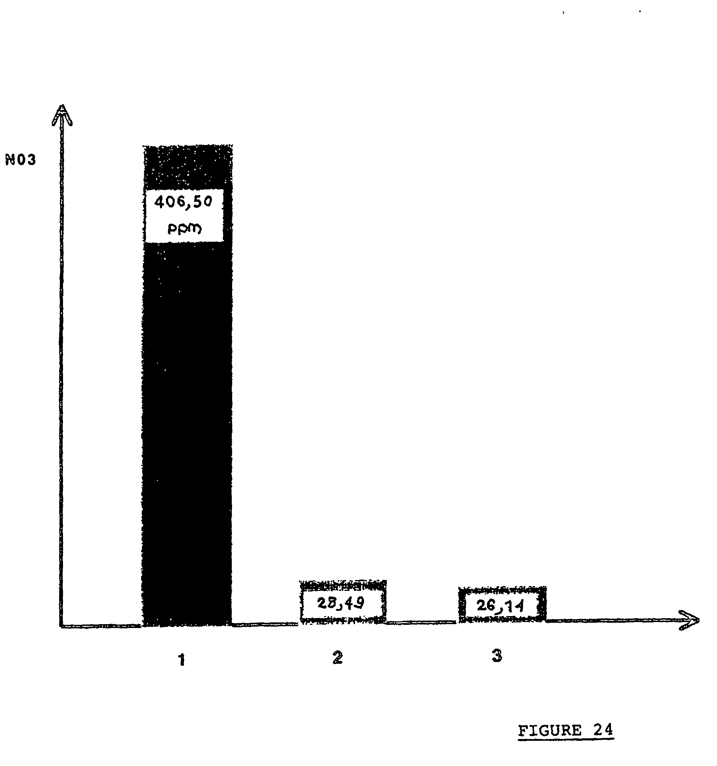

La figure 24 illustre plus précisément l'évolution de la teneur en nitrates de fumiers, selon le traitement auquel ils ont été soumis.

La colonne 1 correspond à du fumier traité par un produit du commerce (BIO-SUPER®), la colonne 2 correspond à une litière en place traitée avec un complexe bactérien selon l'exemple 7, avant compostage, et la colonne 3 illustre les taux de nitrates obtenus sur des litières traitées et compostées conformément à l'invention.

Cette figure montre la capacité des complexes bactériens conformes à l'invention à utiliser les nitrates comme source d'azote.

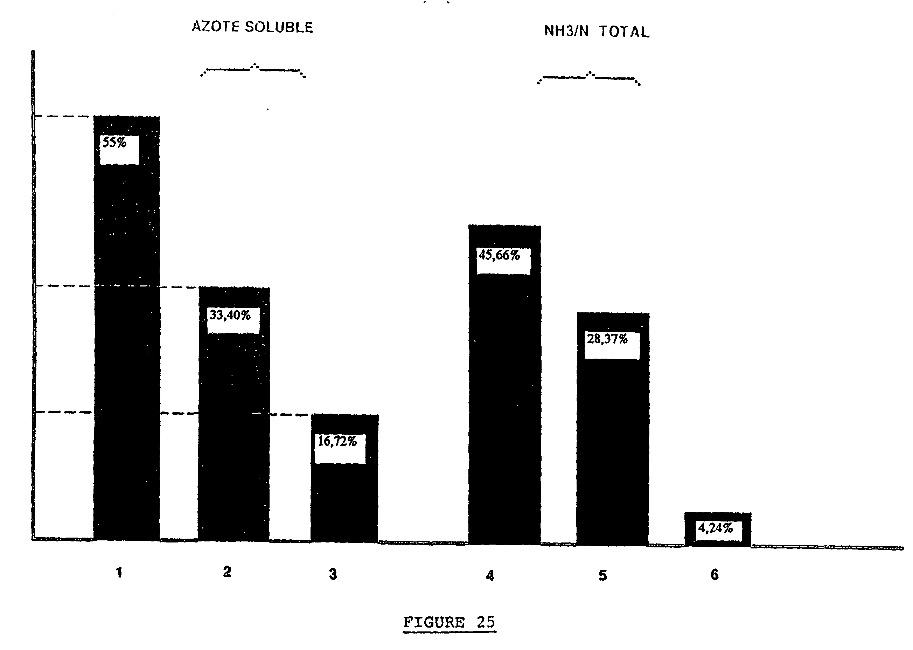

La figure 25 illustre l'intérêt du compostage du fumier pour limiter la pollution des nappes phréatiques et montre, en particulier, la diminution significative d'azote soluble et d'ammoniaque dans un fumier traité et composté conformément à l'invention.

Dans cette figure, les colonnes 1 à 3 illustrent l'évolution de l'azote soluble : la colonne 1 illustre la quantité d'azote soluble (en pourcentage) d'un fumier témoin (55 %), la colonne 2 illustre la quantité d'azote soluble (en pourcentage) d'une litière traitée en place mais non compostée avec un complexe bactérien selon l'exemple 7 (33,4 %) et la colonne 3 illustre la quantité d'azote soluble (en pourcentage) d'une litière traitée et compostée avec un complexe bactérien selon l'exemple 7 (16,72 %) ; les colonnes 4 à 6 illustrent l'évolution du rapport ammoniaque/azote total (en pourcentage) : la colonne 4 illustre ce rapport pour un fumier témoin (45,66 %), ce qui correspond à 83 % de l'azote soluble, la colonne 5 illustre ledit rapport pour une litière traitée en place avec un complexe bactérien selon l'exemple 7 mais non compostée (28,37 %), ce qui correspond à 84,9 % de l'azote soluble et la colonne 6 illustre le même rapport pour une litière traitée et compostée avec un complexe bactérien selon l'exemple 7 (4,24 %), ce qui correspond à 25,4 % de l'azote soluble.

### d) Valeurs et répartition de l'azote dans les fumiers de bovin : étude comparée :

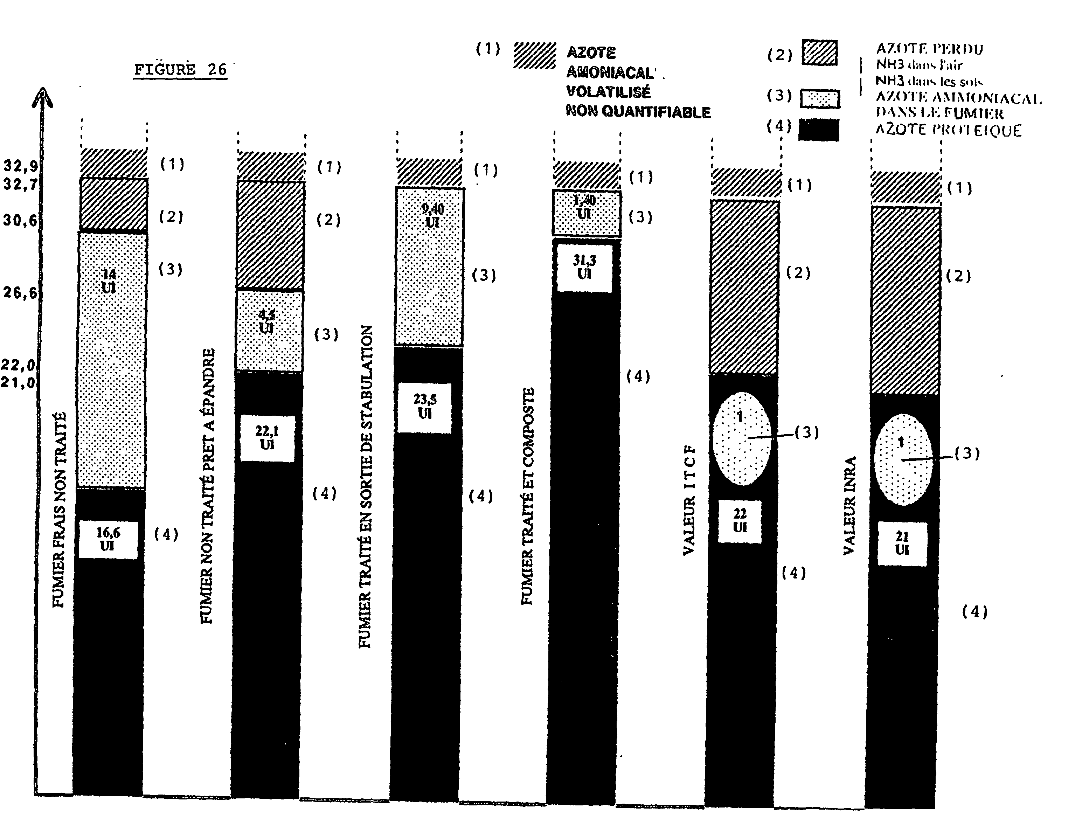

Les figures 26, 27 et 28 montrent l'évolution et la répartition comparées de l'azote : valeurs standards et valeurs obtenues avec un fumier traité conformément à l'invention.

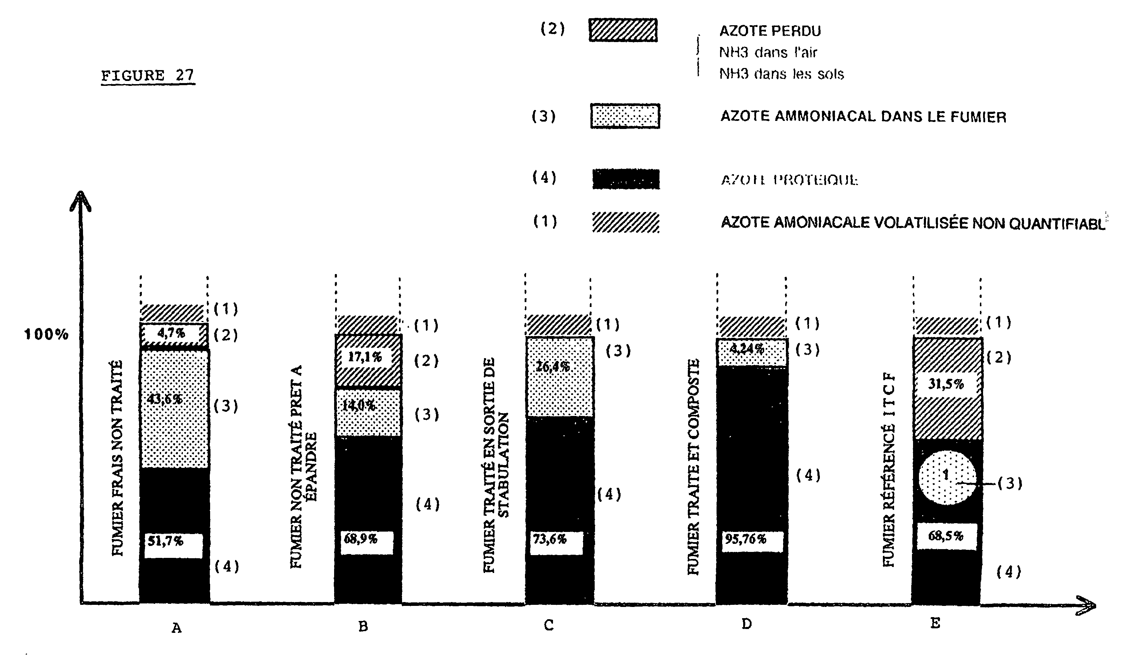

Aux figures 26 et 27, la répartition de l'azote est la suivante :
(1) azote ammoniacal volatilisé non quantifiable,
(2) azote perdu : NH₃ dans l'air, NH₃ dans les sols,
(3) azote ammoniacal dans le fumier,
(4) azote protéique.

A la figure 26, les résultats sont exprimés en valeurs, alors qu'à la figure 27, ils sont exprimés en pourcentage.

Dans ces deux figures, la colonne A correspond à un fumier témoin frais non traité, la colonne B correspond à un fumier témoin non traité prêt à épandre, la colonne C correspond à un fumier traité conformément à l'invention, en sortie de stabulation, la colonne D correspond à un fumier traité et composté conformément à l'invention, les colonnes E (figures 26 et 27) et F (figure 26) sont les valeurs standards fournies par l'ITCF (colonne E) et l'INRA (colonne F).

Ces figures 26 et 27 montrent :
- l'absence d'azote perdu dans un fumier traité conformément à l'invention (colonnes C et D),
- une présence très faible d'azote ammoniacal dans un fumier traité et composté selon l'invention (colonne D) (4,24 % contre 31,5 % pour le fumier ITCF (colonne E)) et une quantité importante d'azote protéique (95,76 %) (colonne D).

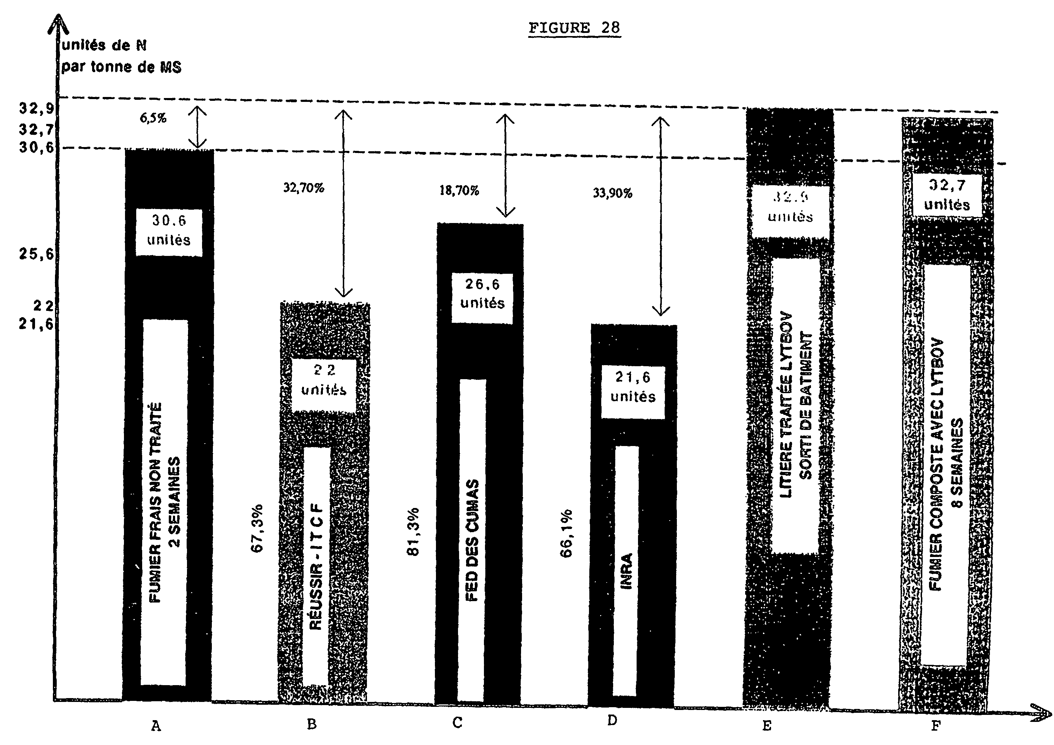

La figure 28 donne les valeurs comparées d'azote/matière sèche : fumier témoin (colonne A), valeurs ITCF (colonnes B et C), valeurs INRA (colonne D) et valeurs selon l'invention (colonnes E et F) et illustre l'absence de perte d'azote dans un fumier traité conformément à l'invention.

## Revendications

1. Complexe bactérien, **caractérisé en ce qu'**il comprend au moins un *Bacillus* non pathogène sélectionné dans le groupe constitué par *B. subtilis, B. amyloliquefaciens, B. megaterium, B. licheniformis* et *B. circulans* et au moins un *Lactobacillus* non pathogène sélectionné dans le groupe constitué par *L. rhamnosus L. paracasei, L. fermentum* et *L. acidophilus,* **en ce qu'**il utilise en tant que source d'azote, de l'azote minéral et des molécules d'azote organique, et **en ce qu'**il est apte à transformer ledit azote en azote organique sous la forme de protéines bactériennes.

2. Complexe bactérien selon la revendication 1, **caractérisé en ce que** ledit azote minéral est sélectionné dans le groupe constitué par l'ammoniaque, les nitrates et les nitrites, et **en ce que** lesdites molécules d'azote organique sont sélectionnées dans le groupe constitué par l'urée, les urates, les aminoacides, les bases azotées et tout autre composé azoté de faible poids moléculaire.

3. Complexe bactérien selon la revendication 1 ou la revendication 2, **caractérisé en ce qu'**il présente au moins les activités enzymatiques suivantes : activité cellulolytique, activité protéolytique, activité amylolytique, activité lipolytique et activité pectinolytique.

4. Complexe bactérien selon l'une quelconque des revendications 1 à 3, **caractérisé en ce que** les proportions *Lactobacillus:Bacillus,* dans ledit complexe, sont comprises entre 100:1 et 1:100.

5. Complexe bactérien selon la revendication 4, **caractérisé en ce que** les proportions *Lactobacillus:Bacillus,* dans ledit complexe, sont comprises entre 10:1 et 1:10.

6. Complexe bactérien selon l'une quelconque des revendications 1 à 5, **caractérisé en ce que** lorsque ledit complexe bactérien comprend plusieurs *Bacillus* et/ou plusieurs *Lactobacillus,* les différentes souches d'un même genre (*Bacillus* ou *Lactobacillus*) sont dans un rapport compris entre 1:1 et 1:100.

7. Complexe bactérien selon l'une quelconque des revendications 1 à 6, **caractérisé en ce que** les concentrations en bactéries sont comprises entre 10² et 10⁸ ufc/g.

8. Complexe bactérien selon l'une quelconque des revendications 1 à 7, **caractérisé en ce qu'**il comprend au moins un *Bacillus* sélectionné dans le groupe constitué par *B. subtilis, B. amyloliquefaciens, B. megaterium, B. licheniformis* et *B. circulans,* à une concentration comprise entre 10² et 10⁷ ufc/g, et au moins un *Lactobacillus* sélectionné dans le groupe constitué par *L. rhamnosus, L. paracasei, L. fermentum* et *L. acidophilus,* à une concentration comprise entre 10³ et 10⁸ ufc/g.

9. Complexe bactérien selon la revendication 8, **caractérisé en ce que** ledit complexe bactérien comprend *B. subtilis* à une concentration comprise entre 10² et 10⁷ ufc/g et un *Lactobacillus* sélectionné dans le groupe constitué par *L. rhamnosus, L. paracasei, L. fermentum* et *L. acidophilus,* à une concentration comprise entre 10³ et 10⁸ ufc/g.

10. Complexe bactérien selon la revendication 8, **caractérisé en ce qu'**il comprend les 5 *Bacillus* suivants : *B. subtilis, B. amyloliquefaciens, B. megaterium, B. licheniformis* et *B. circulans,* chacun à une concentration comprise entre 10² et 10⁷ ufc/g, et les 4 *Lactobacillus* suivants : *L. rhamnosus, L. paracasei,* *L. fermentum* et *L. acidophilus,* chacun à une concentration comprise entre 10³ et 10⁸ ufc/g.

11. Complexe bactérien selon l'une quelconque des revendications 1 à 7, **caractérisé en ce qu'**il comprend au moins un *Bacillus* utilisant, comme source d'azote, les urates.

12. Complexe bactérien selon la revendication 11, **caractérisé en ce que** ledit *Bacillus* est *B. subtilis,* présent à une concentration minimale de 10³ ufc/g.

13. Complexe bactérien selon la revendication 11 ou la revendication 12, **caractérisé en ce qu'**il comprend un autre *Bacillus,* sélectionné dans le groupe constitué par *B. amyloliquefaciens, B. megaterium, B. licheniformis et B. circulans* et au moins un *Lactobacillus* sélectionné dans le groupe constitué par *L. rhamnosus, L. paracasei, L. fermentum, L. acidophilus,* à une concentration comprise entre 10² et 10⁸ ufc/g.

14. Complexe bactérien selon l'une quelconque des revendications 1 à 7, **caractérisé en ce qu'**il comprend au moins un *Lactobacillus* sélectionné dans le groupe constitué par *L. rhamnosus, L. paracasei, L*. *fermentum* et *L. acidophilus* et au moins un *Bacillus* sélectionné dans le groupe constitué par *B. subtilis, B. amyloliquefaciens, B. megaterium, B. licheniformis et B. circulans,* le rapport *Lactobacillus:Bacillus* étant compris entre 1:1 et 1:10.

15. Complexe bactérien selon l'une quelconque des revendications 1 à 7, **caractérisé en ce qu'**il comprend les 2 *Bacillus* suivants : *B. subtilis et B. megaterium,* chacun à une concentration comprise entre 10² et 10⁷ ufc/g, et au moins un *Lactobacillus* sélectionné dans le groupe constitué par *L. rhamnosus, L. paracasei, L. fermentum* et *L. acidophilus,* à une concentration comprise entre 10³ et 10⁸ ufc/g.

16. Complexe bactérien selon l'une quelconque des revendications 1 à 15, **caractérisé en ce qu'**il comprend au moins un *Bacillus* non pathogène, au moins un *Lactobacillus* non pathogène et un *Pediococcus* non pathogène, présent aux mêmes concentrations que les *Lackobacillus.*

17. Complexe bactérien selon l'une quelconque des revendications 1 à 16, **caractérisé en ce qu'**il est associé à au moins un diluant neutre.

18. Complexe bactérien selon la revendication 17, **caractérisé en ce qu'**il est associé, en outre, à un traceur chimique ou microbiologique.

19. Complexe bactérien selon l'une quelconque des revendications précédentes, **caractérisé en ce que** les souches de *Bacillus* sont des souches à Gram+ qui présentent les caractéristiques suivantes :
- elles utilisent, comme source d'azote, de l'azote minéral et des molécules d'azote organique,
- elles présentent au moins l'une des activités suivantes : activité amylolytique, activité cellulolytique, activité lignocellulolytique, activité pectinolytique, activité lipolytique, activité protéolytique, activité kératolytique, activité de type bactériocine ou bactériocine-like, et
- elles présentent au moins les caractéristiques biochimiques suivantes : gélatinase +, catalase +, uréase -, oxydase - et indole -.

20. Complexe bactérien selon la revendication 19, **caractérisé en ce que** lesdites souches de *Bacillus* ont été déposées auprès de la Collection Nationale de Cultures de Micro-organismes (CNCM), en date du 8 juin 1994, sous les numéros I-1433, I-1438 et I-1440 pour ce qui concerne *Bacillus subtilis,* sous les numéros I-1434 et I-1435 pour ce qui concerne *Bacillus* *amyloliquefaciens,* sous le numéro I-1436 pour ce qui concerne *Bacillus megaterium,* sous le numéro I-1437 pour ce qui concerne *Bacillus licheniformis,* et sous le numéro I-1439 pour ce qui concerne *Bacillus circulans.*

21. Complexe bactérien selon l'une quelconque des revendications 16 à 18, **caractérisé en ce que** les souches de *Lactobacillus* sont des souches à Gram + qui présentent les caractéristiques suivantes :
- elles utilisent, comme source d'azote, de l'azote minéral et des molécules d'azote organique,
- elles présentent au moins une activité de type bactériocine ou bactériocine-like, et
- elles présentent au moins les caractéristiques biochimiques suivantes : catalase - et oxydase -.

22. Complexe bactérien selon l'une quelconque des revendications 19 à 21, **caractérisé en ce que** ledit azote minéral est sélectionné dans le groupe constitué par l'ammoniaque, les nitrates et les nitrites, et **en ce que** lesdites molécules d'azote organique sont sélectionnées dans le groupe constitué par l'urée, l'urate, les amino-acides, les bases azotées et tout autre composé azoté de faible poids moléculaire.

23. Complexe bactérien selon la revendication 21 ou la revendication 22, **caractérisé en ce que** lesdites souches de *Lactobacillus* ont été déposées auprès de la Collection Nationale de Cultures de Micro-organismes (CNCM), en date du 28 juillet 1994, sous les numéros I-1450, I-1452, I-1453, I-1454, I-1455, I-1456, I-1459 pour ce qui concerne *Lactobacillus rhamnosus,* sous les numéros I-1451, I-1457 et I-1458 pour ce qui concerne *Lactobacillus paracasei,* sous le numéro I-1460 pour ce qui concerne *Lactobacillus acidophilus,* et sous le numéro I-1461 pour ce qui concerne *Lactobacillus fermentum.*

24. Complexe bactérien selon l'une quelconque des revendications 16 à 18, **caractérisé en ce que** les souches de *Pediococcus* sont des souches à Gram positif qui présentent les caractéristiques suivantes :
- elles présentent une activité de type bactériocine ou bactériocine-like au moins vis-à-vis de *Staphylococcus aureus* et,
- elles présentent au moins les caractéristiques biochimiques suivantes : catalase - et oxydase-.

25. Complexe bactérien selon la revendication 24, **caractérisé en ce que** ladite souche de *Pediococcus* est *Pediococcus pentosaceus,* déposée auprès de la Collection Nationale de Cultures de Micro-organismes (CNCM), en date du 22 décembre 1995, sous le numéro I-1654.

26. Complexe bactérien selon l'une quelconque des revendications 19 à 25, **caractérisé en ce que** les souches de *Bacillus,* de *Lactobacillus* et de *Pediococcus* sont aérobies/anaérobies facultatives.

27. Composition de traitement des résidus biologiques, **caractérisée en ce qu'**elle comprend en association un complexe bactérien selon l'une quelconque des revendications 1 à 18, au moins un diluant neutre et au moins un fixateur de microparticules.

28. Composition du produit dilué prêt à l'emploi selon la revendication 27, **caractérisée en ce qu'**elle comprend essentiellement 5-15 % de complexe bactérien, 80-89 % de diluant neutre et 3-5 % de fixateur de microparticules.

29. Composition selon la revendication 27 ou la revendication 28, **caractérisée en ce qu'**elle comprend, en outre, un traceur chimique ou microbiologique.

30. Procédé de traitement de résidus biologiques, **caractérisé en ce qu'**il comprend la mise en contact d'un complexe bactérien selon l'une quelconque des revendications 1 à 18 ou d'une composition selon l'une quelconque des revendications 27 à 29, avec un résidu biologique à traiter.

31. Procédé selon la revendication 30, **caractérisé en ce que** lesdits résidus biologiques sont des déjections, des litières, du fumier, des lisiers, des cadavres, des lagunes ou des mélanges de déchets végétaux.

32. Procédé de préparation d'un complexe bactérien selon l'une quelconque des revendications 1 à 18, **caractérisé en ce qu'**il comprend :
- la culture et la production d'un nombre présélectionné de souches telles que définies dans l'une quelconque des revendications 19 à 26,
- l'obtention de différentes cultures ayant chacune une concentration en micro-organismes de l'ordre de 10¹⁰-10¹¹ ufc/g,
- la lyophilisation de chaque culture,
- la dilution entre 1/100 et 1/1 000 000 de chacune desdites cultures de souche lyophilisée, en présence d'un diluant neutre, et
- le mélange des différentes souches ainsi diluées pour l'obtention d'un complexe bactérien selon l'une quelconque des revendications 1 à 18.

33. Procédé de préparation d'une composition selon l'une quelconque des revendications 27 à 29, **caractérisé en ce qu'**il comprend le mélange d'un complexe selon l'une quelconque des revendications 1 à 18 avec au moins un diluant neutre et au moins un fixateur de microparticules.

34. Application du complexe bactérien selon l'une quelconque des revendications 1 à 18 ou de la composition selon l'une quelconque des revendications 27 à 29 à la production de produits de transformation non polluants de résidus biologiques.

35. Application du complexe bactérien selon l'une quelconque des revendications 8 à 10 ou 16 à 18, ou d'une composition selon l'une quelconque des revendications 27 à 29 contenant ledit complexe bactérien au traitement des litières de ruminants, d'équidés ou de porcs.

36. Application du complexe bactérien selon l'une quelconque des revendications 11 à 13 ou 16 à 18, ou d'une composition selon l'une quelconque des revendications 27 à 29 contenant ledit complexe bactérien, au traitement des litières de volailles ou d'autres monogastriques.

37. Application du complexe bactérien selon l'une quelconque des revendications 14 ou 16 à 18 ou d'une composition selon l'une quelconque des revendications 27 à 29 contenant ledit complexe bactérien, au traitement des lisiers, des lagunes ou des fosses septiques.

38. Application du complexe bactérien selon l'une quelconque des revendications 15 à 18 ou d'une composition selon l'une quelconque des revendications 27 à 29 contenant ledit complexe bactérien, au traitement des cadavres d'animaux.

39. Application du complexe bactérien selon l'une quelconque des revendications 1 à 13 ou 16 à 18 ou d'une composition selon l'une quelconque des revendications 27 à 29 contenant ledit complexe bactérien, au traitement des produits à composter.

40. Application du complexe bactérien selon l'une quelconque des revendications 1 à 18 ou d'une composition selon l'une quelconque des revendications 27 à 29 contenant ledit complexe bactérien, à la désinfection des locaux.

## Claims

1. Bacterial complex, **characterized in that** it comprises at least one non-pathogenic *Bacillus* chosen from the group consisting of *B. subtilis, B. amyloliquefaciens, B. megaterium, B. licheniformis* and *B. circulans,* and at least one non-pathogenic *Lactobacillus* chosen from the group consisting of *L. rhamnosus, L. paracasei, L. fermentum* and *L. acidophilus,* and **in that** it uses inorganic nitrogen and molecules of organic nitrogen as a nitrogen source, and **in that** it is capable of converting the said nitrogen into organic nitrogen in the form of bacterial proteins.

2. Bacterial complex according to Claim 1, **characterized in that** the said inorganic nitrogen is chosen from the group consisting of ammonia, nitrates and nitrites, and **in that** the said molecules of organic nitrogen are chosen from the group consisting of urea, urates, amino-acids, nitrogenous bases and any other low molecular weight nitrogen compound.

3. Bacterial complex according to Claim 1 or Claim 2, **characterized in that** it shows at least the following enzymatic activities: cellulolytic activity, proteolytic activity, amylolytic activity, lipolytic activity and pectinolytic activity.

4. Bacterial complex according to any of the Claims 1 to 3, **characterized in that** the proportions of *Lactobacillus : Bacillus* in the said complex lie between 100:1 and 1:100.

5. Bacterial complex according to Claim 4, **characterized in that** the proportions of *Lactobacillus : Bacillus* in the said complex lie between 10:1 and 1:10.

6. Bacterial complex according to any of the Claims 1 to 5, **characterized in that** when the said bacterial complex comprises several *Bacillus* and/or several *Lactobacillus,* the different strains of the same species (*Bacillus* or *Lactobacillus*) are in a ratio lying between 1:1 and 1:100.

7. Bacterial complex according to any of the Claims 1 to 6, **characterized in that** the concentrations of bacteria lie between 10² and 10⁸ cfu(colony-forming units)/g.

8. Bacterial complex according to any of the Claims 1 to 7, **characterized in that** it comprises at least one *Bacillus* chosen from the group consisting of *B. subtilis, B. amyloliquefaciens, B. megaterium, B. licheniformis* and *B. circulans* at a concentration lying between 10² and 10⁷ cfu(colony-forming units)/g, and at least one *Lactobacillus* chosen from the group consisting of *L. rhamnosus, L. paracasei, L. fermentum* and *L. acidophilus* at a concentration lying between 10³ and 10⁸ cfu(colony-forming units)/g.

9. Bacterial complex according to Claim 8, **characterized in that** the said bacterial complex comprises *B. subtilis* at a concentration lying between 10² and 10⁷ cfu(colony-forming units)/g, and one *Lactobacillus* chosen from the group consisting of *L. rhamnosus, L. paracasei, L. fermentum* and *L. acidophilus* at a concentration lying between 10³ and 10⁸ cfu(colony-forming units)/g.

10. Bacterial complex according to Claim 8, **characterized in that** it comprises the following 5 *Bacillus: B. subtilis, B. amyloliquefaciens, B. megaterium, B. licheniformis* and *B. circulans* each at a concentration lying between 10² and 10⁷ cru(colony-forming units)/g, and the following 4 *Lactobacillus: L. rhamnosus, L. paracasei, L. fermentum* and *L. acidophilus* each at a concentration lying between 10³ and 10⁸ cfu(colony-forming units)/g.

11. Bacterial complex according to any of the Claims 1 to 7, **characterized in that** it comprises at least one *Bacillus* using urates as the source of nitrogen.

12. Bacterial complex according to Claim 11 **characterized in that** the said *Bacillus* is *B. subtilis* present at a minimum concentration of 10³ cfu(colony-forming units)/g..

13. Bacterial complex according to Claim 11 or Claim 12, **characterized in that** it comprises another *Bacillus* chosen from the group consisting of *B. amyloliquefaciens. B. megaterium, B. licheniformis* and *B. circulans,* and at least one *Lactobacillus* chosen from the group consisting of *L. rhamnosus, L. paracasei, L. fermentum* and *L. acidophilus* at a concentration lying between 10² and 10⁸ cru(colony-forming units)/g.

14. Bacterial complex according to any of the Claims 1 to 7, **characterized in that** it comprises at least one *Lactobacillus* chosen from the group consisting of *L. rhamnosus, L. paracasei, L. fermentum* and *L. acidophilus* and at least one *Bacillus* chosen from the group consisting of *B. subtilis, B. amyloliquefaciens, B. megaterium, B. licheniformis* and *B. circulans,* the ratio of *Lactobacillus : Bacillus* lying between 1:1 and 1:10.

15. Bacterial complex according to any of the Claims 1 to 7, **characterized in that** it comprises the following 2 *Bacillus: B. subtilis* and *B. megaterium,* each at a concentration lying between 10² and 10⁷ cfu(colony-forming units)/g, and at least one *Lactobacillus* chosen from the group consisting of *L. rhamnosus, L. paracasei, L. fermentum* and *L. acidophilus* at a concentration lying between 10³ and 10⁸ cfu(colony-forming units)/g.

16. Bacterial complex according to any of the Claims 1 to 15, **characterized in that** it comprises at least one non-pathogenic *Bacillus,* at least one non-pathogenic *Lactobacillus* and one non-pathogenic *Pediococcus* present at the same concentrations as the *Lactobacillus.*

17. Bacterial complex according to any of the Claims 1 to 16, **characterized in that** it is associated with at least one neutral (inert) diluent.

18. Bacterial complex according to Claim 17, **characterized in that** it is additionally associated with at least one chemical or microbiological tracer.

19. Bacterial complex according to any of the foregoing Claims, **characterized in that** the strains of *Bacillus* are Gram +ve strains showing the following characteristics:
- they use inorganic nitrogen and molecules of organic nitrogen as a nitrogen source,
- they show at least one of the following activities: amylolytic activity, cellulolytic activity, lignocellulolytic activity, pectinolytic activity, lipolytic activity, proteolytic activity, keratinolytic activity, activity of the bacteriocin or bacteriocin-like type, and
- they show at least the following biochemical characteristics: gelatinase +, catalase +, urease -, oxidase - and indole -.

20. Bacterial complex according to Claim 19, **characterized in that** the said strains of *Bacillus* were deposited with the National Collection of Micro-organism Cultures (CNCM), dated 8^{th} June 1994, under numbers I-1433, I-1438 and I-1440 with regard to *Bacillus subtilis,* under numbers I-1434 and I-1435 with regard to *Bacillus amyloliquefaciens,* under number I-1436 with regard to *Bacillus megaterium,* under number I-1437 with regard to *Bacillus licheniformis* and under number I-1439 with regard to *Bacillus circulans.*

21. Bacterial complex according to any of the Claims 16 to 18, **characterized in that** the strains of *Lactobacillus* are Gram +ve strains showing the following characteristics:
- they use inorganic nitrogen and molecules of organic nitrogen as a nitrogen source,
- they show at least one activity of the bacteriocin or bacteriocin-like type, and
- they show at least the following biochemical characteristics: catalase - and oxidose -.

22. Bacterial complex according to any of the Claims 19 to 21, **characterized in that** the said inorganic nitrogen is chosen from the group consisting of ammonia, nitrates and nitrites, and **in that** the said molecules of organic nitrogen are chosen from the group consisting of urea, urate, amino-acids, nitrogenous bases and any other low molecular weight nitrogen compound.

23. Bacterial complex according to Claim 21 or Claim 22, **characterized in that** the said strains of *Lactobacillus* were deposited with the National Collection of Micro-organism Cultures (CNCM), dated 28^{th} July 1994, under numbers I-1450, I-1452, I-1453, I-1454, I-1455, I-1456 and I-1459 with regard to *Lactobacillus rhamnosus,* under numbers I-1451, I-1457 and I-1458 with regard to *Lactobacillus paracasei,* under number I-1460 with regard to *Lactobacillus acidophilus* and under number I-1461 with regard to *Lactobacillus fermentum.*

24. Bacterial complex according to any of the Claims 16 to 18, **characterized in that** the strains of *Pediococcus* are Gram positive strains showing the following characteristics:
- they show one activity of the bacteriocin or bacteriocin-like type at least towards *Staphylococcus aureus,* and
- they show at least the following biochemical characteristics: catalase - and oxidase -.

25. Bacterial complex according to Claim 24, **characterized in that** the said strain of *Pediococcus* is *Pediococcus pentosaceus,* deposited with the National Collection of Micro-organism Cultures (CNCM), dated 22^{nd} December 1995, under number I-1654.

26. Bacterial complex according to any of the Claims 19 to 25, **characterized in that** the strains of *Bacillus,* of *Lactobacillus* and of *Pediococcus* are facultative aerobes/anaerobes.

27. Composition for treating biological residues, **characterized in that** it comprises, in association, a bacterial complex according to any of the Claims 1 to 18, at least one neutral (inert) diluent and at least one micro-particles fixing agent.

28. Composition of diluted product ready for use according to Claim 27, **characterized in that** it comprises essentially 5 - 15 % of the bacterial complex, 80 - 89 % of the neutral (inert) diluent and 3 - 5 % of the micro-particles fixing agent.

29. Composition according to Claim 27 or Claim 28, **characterized in that** it additionally comprises a chemical or microbiological tracer.

30. Process for treating biological residues, **characterized in that** it comprises contacting a bacterial complex according to any of the Claims 1 to 18 or a composition according to any of the Claims 27 to 29 with a biological residue to be treated.

31. Process according to Claim 30, **characterized in that** the said biological residues are dejecta, litter, manure, liquid manure (dung slurry), animal carcasses, lagoons or mixtures of vegetable wastes.

32. Process for preparing a bacterial complex according to any of the Claims 1 to 18, **characterized in that** it comprises:
- the culturing and production of a pre-selected number of strains as defined in any of the Claims 19 to 26,
- obtaining different cultures each having a concentration of micro-organisms of the order of 10¹⁰ - 10¹¹ cfu(colony-forming units)/g,
- lyophilising (freeze-drying) each culture,
- the dilution between 1/100 and 1/1,000,000 of each of the said cultures of lyophilised strain in the presence of a neutral (inert) diluent, and
- mixing together the different strains thus diluted to obtain a bacterial complex according to any of the Claims 1 to 18.

33. Process for preparing a composition according to any of the Claims 27 to 29, **characterized in that** it comprises mixing a complex according to any of the Claims 1 to 18 with at least one neutral (inert) diluent and at least one micro-particles fixing agent.

34. Use of the bacterial complex according to any of the Claims 1 to 18 or of the composition according to any of the Claims 27 to 29 to the production of non-polluting conversion products from biological residues.

35. Use of the bacterial complex according to any of the Claims 8 to 10 or 16 to 18, or of a composition according to any of the Claims 27 to 29, containing the said bacterial complex for the treatment of the litter of ruminants, Equidae or pigs.

36. Use of the bacterial complex according to any of the Claims 11 to 13 or 16 to 18, or of a composition according to any of the Claims 27 to 29, containing the said bacterial complex for the treatment of the litter of poultry or other monogastrics.

37. Use of the bacterial complex according to any of the Claims 14 or 16 to 18, or of a composition according to any of the Claims 27 to 29, containing the said bacterial complex for the treatment of liquid manure, lagoons or septic tanks.

38. Use of the bacterial complex according to any of the Claims 15 to 18, or of a composition according to any of the Claims 27 to 29, containing the said bacterial complex for the treatment of animal carcasses.

39. Use of the bacterial complex according to any of the Claims 1 to 13 or 16 to 18, or of a composition according to any of the Claims 27 to 29, containing the said bacterial complex for the treatment of products to be composted.

40. Use of the bacterial complex according to any of the Claims 1 to 18, or of a composition according to any of the Claims 27 to 29, containing the said bacterial complex for the disinfection of premises.

## Patentansprüche

1. Bakterienkomplex, **dadurch gekennzeichnet, daß** er wenigstens einen nichtpathogenen *Bacillus,* ausgewählt aus der Gruppe bestehend aus *B. subtilis, B. amyloliquefaciens, B. megaterium, B. licheniformis* und *B. circulans,* und wenigstens einen nichtpathogenen *Lactobacillus,* ausgewählt aus der Gruppe bestehend aus *L. rhamnosus, L. paracasei, L. fermentum* und *L. acidophilus,* umfaßt, daß er als Stickstoffquelle anorganischen Stickstoff und organische Stickstoffmoleküle verwertet, und daß er fähig ist, diesen Stickstoff in organischen Stickstoff in Form von bakteriellen Proteinen umzuwandeln.

2. Bakterienkomplex nach Anspruch 1, **dadurch gekennzeichnet, daß** der anorganische Stickstoff ausgewählt ist aus der Gruppe, die aus Ammoniak, Nitraten und Nitriten besteht, und daß die organischen Stickstoffmoleküle ausgewählt sind aus der Gruppe, die aus Harnstoff, Uraten, Aminosäuren, Stickstoffbasen und jeder anderen niedermolekularen Stickstoffverbindung besteht.

3. Bakterienkomplex nach Anspruch 1 oder Anspruch 2, **dadurch gekennzeichnet, daß** er wenigstens die folgenden enzymatischen Aktivitäten aufweist: cellulolytische Aktivität, proteolytische Aktivität, amylolytische Aktivität, lipolytische Aktivität und pektinolytische Aktivität.

4. Bakterienkomplex nach irgendeinem der Ansprüche 1 bis 3, **dadurch gekennzeichnet, daß** das Verhältnis von *Lactobacillus : Bacillus* in dem Komplex zwischen 100:1 und 1:100 liegt.

5. Bakterienkomplex nach Anspruch 4, **dadurch gekennzeichnet, daß** das Verhältnis von *Lactobacillus : Bacillus* in dem Komplex zwischen 10:1 und 1:10 liegt.

6. Bakterienkomplex nach irgendeinem der Ansprüche 1 bis 5, **dadurch gekennzeichnet, daß** wenn der Bakterienkomplex mehrere *Bacillus-* und/oder mehrere *Lactobacillus*-Stämme umfaßt, die verschiedenen Stämme einer selben Gattung *(Bacillus* oder *Lactobacillus)* in einem Verhältnis zwischen 1:1 und 1:100 vorliegen.

7. Bakterienkomplex nach irgendeinem der Ansprüche 1 bis 6, **dadurch gekennzeichnet, daß** die Bakterienkonzentrationen zwischen 10² und 10⁸ CFU/g liegen.

8. Bakterienkomplex nach irgendeinem der Ansprüche 1 bis 7, **dadurch gekennzeichnet, daß** er wenigstens einen *Bacillus,* ausgewählt aus der Gruppe bestehend aus B. *subtilis, B. amyloliquefaciens, B. megaterium, B. licheniformis* und *B. circulans,* in einer Konzentration zwischen 10² und 10⁷ CFU/g und wenigstens einen *Lactobacillus,* ausgewählt aus der Gruppe bestehend aus *L. rhamnosus, L. paracasei, L. fermentum* und *L. acidophilus,* in einer Konzentration zwischen 10³ und 10⁸ CFU/g umfaßt.

9. Bakterienkomplex nach Anspruch 8, **dadurch gekennzeichnet, daß** der Bakterienkomplex *B. subtilis* in einer Konzentration zwischen 10² und 10⁷ CFU/g und einen *Lactobacillus,* ausgewählt aus der Gruppe bestehend aus *L. rhamnosus, L. paracasei, L. fermentum* und *L. acidophilus,* in einer Konzentration zwischen 10³ und 10⁸ CFU/g umfaßt.

10. Bakterienkomplex nach Anspruch 8, **dadurch gekennzeichnet, daß** er die fünf folgenden *Bacillus-Arten: B. subtilis, B. amyloliquefaciens, B. megaterium, B. licheniformis* und *B. circulans,* jeweils in einer Konzentration zwischen 10² und 10⁷ CFU/g, und die vier folgenden *Lactobacillus*-Arten: *L. rhamnosus, L. paracasei, L. fermentum* und *L. acidophilus,* jeweils in einer Konzentration zwischen 10³ und 10⁸ CFU/g, umfaßt.

11. Bakterienkomplex nach irgendeinem der Ansprüche 1 bis 7, **dadurch gekennzeichnet, daß** er wenigstens einen *Bacillus* umfaßt, der als Stickstoffquelle Urate verwertet.

12. Bakterienkomplex nach Anspruch 11, **dadurch gekennzeichnet, daß** der *Bacillus B. subtilis* ist, der in einer Minimalkonzentration von 10³ CFU/g vorliegt.

13. Bakterienkomplex nach Anspruch 11 oder Anspruch 12, **dadurch gekennzeichnet, daß** er einen weiteren *Bacillus,* ausgewählt aus der Gruppe bestehend aus *B. amyloliquefaciens, B. megaterium, B. licheniformis* und *B. circulans,* und wenigstens einen *Lactobacillus,* ausgewählt aus der Gruppe bestehend aus *L. rhamnosus, L. paracasei, L. fermentum* und *L. acidophilus,* in einer Konzentration zwischen 10² und 10⁸ CFU/g umfaßt.

14. Bakterienkomplex nach irgendeinem der Ansprüche 1 bis 7, **dadurch gekennzeichnet, daß** er wenigstens einen *Lactobacillus,* ausgewählt aus der Gruppe bestehend aus *L. rhamnosus, L. paracasei, L. fermentum* und *L. acidophilus,* und wenigstens einen *Bacillus,* ausgewählt aus der Gruppe bestehend aus *B. subtilis, B. amyloliquefaciens, B. megaterium, B. licheniformis* und *B. circulans,* umfaßt, wobei das Verhältnis von *Lactobacillus : Bacillus* zwischen 1:1 und 1:10 liegt.

15. Bakterienkomplex nach irgendeinem der Ansprüche 1 bis 7, **dadurch gekennzeichnet, daß** er die beiden folgenden *Bacillus*-Arten: *B. subtilis,* und *B. megaterium,* jeweils in einer Konzentration zwischen 10² und 10⁷ CFU/g, und wenigstens einen *Lactobacillus,* ausgewählt aus der Gruppe bestehend aus *L. rhamnosus, L. paracasei, L.* *fermentum* und *L. acidophilus,* jeweils in einer Konzentration zwischen 10³ und 10⁸ CFU/g, umfaßt.

16. Bakterienkomplex nach irgendeinem der Ansprüche 1 bis 15, **dadurch gekennzeichnet, daß** er wenigstens einen nichtpathogenen *Bacillus,* wenigstens einen nichtpathogenen *Lactobacillus* und einen nichtpathogenen *Pediococcus* umfaßt, der in der gleichen Konzentration wie *Lactobacillus* vorliegt.

17. Bakterienkomplex nach irgendeinem der Ansprüche 1 bis 16, **dadurch gekennzeichnet, daß** er mit wenigstens einem neutralen Verdünnungsmittel assoziiert ist.

18. Bakterienkomplex nach Anspruch 17, **dadurch gekennzeichnet, daß** er ferner mit einem chemischen oder mikrobiologischen Tracer assoziiert ist.

19. Bakterienkomplex nach irgendeinem der vorhergehenden Ansprüche, **dadurch gekennzeichnet, daß** die *Bacillus*-Stämme Gram+ -Stämme sind, die die folgenden Eigenschaften aufweisen:
- sie verwerten als Stickstoffquelle anorganischen Stickstoff und organische Stickstoffmoleküle,
- sie weisen wenigstens eine der folgenden Aktivitäten auf: amylolytische Aktivität, cellulolytische Aktivität, lignocellulolytische Aktivität, pektinolytische Aktivität, lipolytische Aktivität, proteolytische Aktivität, keratolytische Aktivität, Bakteriozin- oder Bakteriozin-ähnliche Aktivität, und
- sie weisen wenigstens die folgenden biochemischen Charakteristka auf: Gelatinase +, Katalase +, Urease -, Oxidase - und Indol -.

20. Bakterienkomplex nach Anspruch 19, **dadurch gekennzeichnet, daß** die *Bacillus-*Stämme bei der Collection Nationale de Cultures de Microorganismes (CNCM) am 8. Juni 1994 unter den Nummern I-1433, I-1438 und I-1440 für *Bacillus subtilis,* unter den Nummern I-1434 und I-1435 für *Bacillus amyloliquefaciens,* unter der Nummer I-1436 für *Bacillus megaterium,* unter der Nummer I-1437 für *Bacillus licheniformis,* und unter der Nummer I-1439 für *Bacillus circulans* hinterlegt wurden.

21. Bakterienkomplex nach irgendeinem der Ansprüche 16 bis 18, **dadurch gekennzeichnet, daß** die *Lactobacillus*-Stämme Gram+ -Stämme sind, die die folgenden Eigenschaften aufweisen:
- sie verwerten als Stickstoffquelle anorganischen Stickstoff und organische Stickstoffmoleküle,
- sie weisen wenigstens eine Bakteriozin- oder Bakteriozin-ähnliche Aktivität auf, und
- sie weisen wenigstens die folgenden biochemischen Charakteristika auf: Katalase - und Oxidase -.

22. Bakterienkomplex nach irgendeinem der Ansprüche 19 bis 21, **dadurch gekennzeichnet, daß** der anorganische Stickstoff ausgewählt ist aus der Gruppe, die aus Ammoniak, Nitraten und Nitriten besteht, und daß die organischen Stickstoffmoleküle ausgewählt sind aus der Gruppe, die aus Harnstoff, Urat, Aminosäuren, Stickstoffbasen und jeder anderen niedermolekularen Stickstoffverbindung besteht.

23. Bakterienkomplex nach Anspruch 21 oder Anspruch 22, **dadurch gekennzeichnet, daß** die *Lactobacillus*-Stämme bei der Collection Nationale de Cultures de Microorganismes (CNCM) am 28. Juli 1994 unter den Nummern I-1450, I-1452, I-1453, I-1454, I-1455, I-1456, I-1459 für *Lactobacillus rhamriosus,* unter den Nummern I-1451, I-1457 und I-1458 für *Lactobacillus paracasei*, unter der Nummer I-1460 für *Lactobacillus acidophilus,* und unter der Nummer I-1461 für *Lactobacillus fermentum* hinterlegt wurden.

24. Bakterienkomplex nach irgendeinem der Ansprüche 16 bis 18, **dadurch gekennzeichnet, daß** die *Pediococcus-*Stämme Gram-positive Stämme sind, die die folgenden Eigenschaften aufweisen:
- sie weisen zumindest gegenüber *Staphylococcus aureus* eine Bakteriozin- oder Bakteriozin-ähnliche Aktivität auf, und
- sie weisen wenigstens die folgenden biochemischen Charakteristika auf: Katalase - und Oxidase -.

25. Bakterienkomplex nach Anspruch 24, **dadurch gekennzeichnet, daß** der *Pediococcus*-Stamm *Pediococcus pentosaceus* ist, hinterlegt bei der Collection Nationale de Cultures de Microorganismes (CNCM) am 22. Dezember 1995 unter der Nummer I-1654.

26. Bakterienkomplex nach irgendeinem der Ansprüche 19 bis 25, **dadurch gekennzeichnet, daß** die Stämme von *Bacillus, Lactobacillus* und *Pediococcus* fakultative Aerobier/Anaerobier sind.

27. Zusammensetzung zur Behandlung biologischer Abfälle, **dadurch gekennzeichnet, daß** sie in Assoziation einen Bakterienkomplex nach irgendeinem der Ansprüche 1 bis 18, wenigstens ein neutrales Verdünnungsmittel und wenigstens ein Fixiermittel für Mikropartikel umfaßt.

28. Zusammensetzung des gebrauchsfertigen verdünnten Produkts nach Anspruch 27, **dadurch gekennzeichnet, daß** sie im wesentlichen 5-15% Bakterienkomplex, 80-89% neutrales Verdünnungsmittel und 3-5% Fixiermittel für Mikropartikel umfaßt.

29. Zusammensetzung nach Anspruch 27 oder Anspruch 28, **dadurch gekennzeichnet, daß** sie ferner einen chemischen oder mikrobiologischen Tracer umfaßt.

30. Verfahren zur Behandlung biologischer Abfälle, **dadurch gekennzeichnet, daß** es das Inkontaktbringen eines Bakterienkomplexes nach irgendeinem der Ansprüche 1 bis 18 oder einer Zusammensetzung nach irgendeinem der Ansprüche 27 bis 29 mit einem zu behandelnden biologischen Abfall umfaßt.

31. Verfahren nach Anspruch 30, **dadurch gekennzeichnet, daß** die biologischen Abfälle aus Fäkalien, Streu, Stallmist, Gülle, Kadavern, Lagunen oder Mischungen pflanzlicher Abfälle stammen.

32. Verfahren zur Herstellung eines Bakterienkomplexes nach irgendeinem der Ansprüche 1 bis 18, **dadurch gekennzeichnet, daß** es umfaßt:
- Kultivierung und Produktion einer vorgewählten Anzahl von Stämmen, wie sie in irgendeinem der Ansprüche 19 bis 26 definiert sind,
- Erhalt verschiedener Kulturen, von denen jede eine Mikroorganismenkonzentration in einem Bereich von 10¹⁰-10¹¹ CFU/g besitzt,
- Lyophilisierung jeder Kultur,
- Verdünnung zwischen 1/100 und 1/1 000 000 jeder lyophilisierten Stammkultur in Gegenwart eines neutralen Verdünnungsmittels, und
- Vermischen der verschiedenen so verdünnten Stämme zum Erhalt eines Bakterienkomplexes nach irgendeinem der Ansprüche 1 bis 18.

33. Verfahren zur Herstellung einer Zusammensetzung nach irgendeinem der Ansprüche 27 bis 29, **dadurch gekennzeichnet, daß** es das Vermischen eines Komplexes nach irgendeinem der Ansprüche 1 bis 18 mit wenigstens einem neutralen Verdünnungsmittel und wenigstens einem Fixiermittel für Mikropartikel umfaßt.

34. Verwendung des Bakterienkomplexes nach irgendeinem der Ansprüche 1 bis 18 oder der Zusammensetzung nach irgendeinem der Ansprüche 27 bis 29 zur Herstellung nicht umweltschädlicher Transformationsprodukte aus biologischen Abfällen.

35. Verwendung des Bakterienkomplexes nach irgendeinem der Ansprüche 8 bis 10 oder 16 bis 18 oder einer Zusammensetzung nach irgendeinem der Ansprüche 27 bis 29, die den Bakterienkomplex enthält, zur Behandlung der Streu von Wiederkäuern, Einhufern oder Schweinen.

36. Verwendung des Bakterienkomplexes nach irgendeinem der Ansprüche 11 bis 13 oder 16 bis 18 oder einer Zusammensetzung nach irgendeinem der Ansprüche 27 bis 29, die den Bakterienkomplex enthält, zur Behandlung der Streu von Geflügel oder anderen Monogastriden.

37. Verwendung des Bakterienkomplexes nach irgendeinem der Ansprüche 14 oder 16 bis 18 oder einer Zusammensetzung nach irgendeinem der Ansprüche 27 bis 29, die den Bakterienkomplex enthält, zur Behandlung von Gülle, Lagunen oder Klärgruben.

38. Verwendung des Bakterienkomplexes nach irgendeinem der Ansprüche 15 bis 18 oder einer Zusammensetzung nach irgendeinem der Ansprüche 27 bis 29, die den Bakterienkomplex enthält, zur Behandlung von Tierkadavern.

39. Verwendung des Bakterienkomplexes nach irgendeinem der Ansprüche 1 bis 13 oder 16 bis 18 oder einer Zusammensetzung nach irgendeinem der Ansprüche 27 bis 29, die den Bakterienkomplex enthält, zur Behandlung von zu kompostierenden Produkten.

40. Verwendung des Bakterienkomplexes nach irgendeinem der Ansprüche 1 bis 18 oder einer Zusammensetzung nach irgendeinem der Ansprüche 27 bis 29, die den Bakterienkomplex enthält, zur Desinfektion von Räumen.
